# EUROPÄISCHE PATENTANMELDUNG

(11) **EP 2 873 856 A1**
(43) Veröffentlichungstag der Anmeldung: **20.05.2015**
(21) Anmeldenummer: 14188273.8
(22) Anmeldetag: 09.10.2014
(51) Int. Cl.: F04B 9/127, F01B 11/00, F04B 7/04

(54) **Druckgasmotor zum Betreiben eines Lavage-Systems**

(30) Priorität: 18.11.2013 DE 102013223501
(71) Anmelder: Heraeus Medical GmbH, 61273 Wehrheim (DE)
(72) Erfinder: Dr. Vogt, Sebastian, 99092 Erfurt (DE)
(74) Vertreter: Panten, Kirsten

(57) **Zusammenfassung**

Die Erfindung betrifft einen Druckgasmotor (1) aufweisend einen Arbeitskolben (12), einen Innenraum, der an einer Rückseite geschlossen ist und in dem der Arbeitskolben (12) linear beweglich angeordnet ist, ein Rückstellelement (22), das zumindest zeitweise eine in Richtung der Rückseite wirkende Kraft auf den Arbeitskolben (12) ausübt, eine Gaseinlassöffnung (24) zum Einspeisen eines unter Druck stehenden Gases in den Innenraum und eine Gasauslassöffnung (26) zum Abführen des Gases aus dem Innenraum, wobei zwischen dem Arbeitskolben (12) und der geschlossenen Rückseite ein Steuerkolben (14) linear beweglich im Innenraum angeordnet ist, wobei der Steuerkolben (14) in einer ersten Position die Gasauslassöffnung (26) abdeckt und die Gaseinlassöffnung (24) nicht abdeckt und in einer zweiten Position die Gaseinlassöffnung (24) abdeckt und die Gasauslassöffnung (26) nicht abdeckt, der Steuerkolben (14) gegen den Arbeitskolben (12) beweglich gelagert ist und an dem Arbeitskolben (12) und/oder dem Steuerkolben (14) ein Mitnehmerelement (18, 20, 42) angeordnet ist, das bei einer Bewegung des Arbeitskolbens (12) vom Steuerkolben (14) weg den Steuerkolben (14) in Richtung des Arbeitskolbens (12) mit einem ersten Abstand nachzieht sowie ein Lavage-System mit einem solchen Druckgasmotor (1). Die Erfindung betrifft auch ein Verfahren zum Erzeugen einer periodischen Bewegung mit einem Druckgas, bei dem in einem Ausgangszustand der Arbeitskolben (12) und der Steuerkolben (14) in dem Innenraum voneinander mit dem ersten Abstand beabstandet werden, wobei der Steuerkolben (14) die Gasauslassöffnung (26) verschließt und die Gaseinlassöffnung (24) zwischen dem Arbeitskolben (12) und dem Steuerkolben (14) offen liegt; das Druckgas zwischen den Arbeitskolben (12) und den Steuerkolben (14) in den Innenraum geleitet wird; der Arbeitskolben (12) durch den Druck des Druckgases in Richtung einer Vorderseite des Innenraums vom Steuerkolben (14) weg beschleunigt und bewegt wird, wobei sich der Abstand zwischen dem Arbeitskolben (12) und dem Steuerkolben (14) vergrößert; der Steuerkolben (14) über das Mitnehmerelement (18, 20, 42) von dem Arbeitskolben (12) mitgezogen wird, bevorzugt ab Erreichen des zweiten Abstands; der Steuerkolben (14) die Gaseinlassöffnung (24) aufgrund der Bewegung des Steuerkolbens (14) verschließt; der Steuerkolben (14) die Gasauslassöffnung (26) aufgrund der Bewegung des Steuerkolbens (14) öffnet; der Arbeitskolben (12) durch das Rückstellelement (22) in Richtung der Rückseite des Innenraums beschleunigt wird; das Druckgas zwischen dem Arbeitskolben (12) und dem Steuerkolben (14) durch wenigstens eine gasdurchlässige Öffnung durch den Steuerkolben (14) in den Raum zwischen der Rückseite des Steuerkolbens (14) und der Rückwand des Innenraums strömt; das Druckgas in dem Raum zwischen der Rückseite des Steuerkolbens (14) und der Rückwand des Innenraums durch die geöffnete Gasauslassöffnung (26) an die Umgebung abgegeben wird; der Arbeitskolben (12) auf den Steuerkolben (14) trifft und diesen in Richtung der Rückseite des Innenraums bewegt; durch die umgekehrte Bewegung des Steuerkolbens (14) die Gasauslassöffnung (26) wieder geschlossen wird und durch die umgekehrte Bewegung des Steuerkolbens (14) die Gaseinlassöffnung (24) wieder geöffnet wird, so dass erneut Druckgas zwischen den Arbeitskolben (12) und den Steuerkolben (14) in den Innenraum geleitet wird.

## Beschreibung

Die Erfindung betrifft einen Druckgasmotor aufweisend einen Arbeitskolben, einen Innenraum, der an einer Rückseite geschlossen ist und in dem der Arbeitskolben linear beweglich angeordnet ist, ein Rückstellelement, das zumindest zeitweise eine in Richtung der Rückseite wirkende Kraft auf den Arbeitskolben ausübt, eine Gaseinlassöffnung zum Einspeisen eines unter Druck stehenden Gases in den Innenraum und eine Gasauslassöffnung zum Abführen des Gases aus dem Innenraum. Die Erfindung betrifft auch ein Lavage-System mit einem solchen Druckgasmotor.

Die Erfindung betrifft auch die Verwendung eines solchen Druckgasmotors und ein Verfahren zum Erzeugen einer periodischen Bewegung mit einem Druckgas.

Gegenstand der Erfindung ist somit ein vereinfachter Druckgasmotor, der im Wesentlichen aus kostengünstigen Kunststoffen gefertigt werden kann und zum Antrieb von nur einmalig, kurzzeitig betriebenen Vorrichtungen, insbesondere von aus Kunststoffen gefertigten, einmalig einsetzbaren medizinischen Sprühvorrichtungen bestimmt ist. Es wird ferner eine durch den Druckgasmotor angetrieben Pumpe für Flüssigkeiten beschrieben. Weiterhin wird die Verwendung des Druckgasmotors zum Antrieb einer Pumpe zum Austragen von Spülflüssigkeit einer medizinischen Sprühvorrichtung (das heißt eines Lavage-Systems) vorgeschlagen, die zum nur einmaligen Gebrauch bestimmt ist.

In der Chirurgie werden medizinische Spülsysteme in breitem Umfang eingesetzt, um Gewebeareale zu reinigen. Diese Spülsysteme werden als Lavage-Systeme bezeichnet. Mit den Lavage-Systemen werden mit den Spülflüssigkeiten Sprühstrahlen erzeugt, die auf die zu reinigenden Gewebeareale auftreffen und eine mechanische Reinigungswirkung auf diese Gewebeareale ausüben. Insbesondere bei der Implantation von Gelenkendoprothesen und bei septischen Revisionen sind Lavage-Systeme von wesentlicher Bedeutung (R. M. Sherman et al.: The role of lavage in preventing hemodynamic and blood-gas changes during cemented arthroplasty. J. Bone Joint. Surg. 1983; 65-A: 500-506; S. J. Breusch et al.: Zementierte Hüftendoprothetik: Verminderung des Fettembolierisikos in der zementierten Hüftendoprothetik mittels gepulster Druckspülung. Orthopädie 2000; 29: 578-586; S. J. Breusch et al.: Lavage technique in THA: Jet-lavage Produces Better Cement Penetration Than Syringe-Lavage in the Proximal Femur. J. Arthroplasty. 200; 15(7): 921-927; R. J. Byrick et al.: High-volume, high pressure pulsatile lavage during cemented arthroplasty. J. Bone Joint Surg. 1989; 81-A: 1331-1336; J. Christie et al.: Medullary lavage reduces embolic phenomena and cardiopulmonary changes during cemented hemiarthorplasty. J. Bone Joint Surg. 1995; 77-B: 456-459).

Gepulste Lavage-Systeme sind seit langem bekannt, beispielsweise aus der US 4,583,531 A, der US 4,278,078 A und der US 5,542,918 A. Die gegenwärtig im Markt befindlichen Lavage-Systeme werden durch Elektromotoren (zum Beispiel InterPulse^{®} Jet Lavage der Stryker GmbH & Co. KG) oder durch Druckluft (zum Beispiel PALAVAGE^{®} der Heraeus Medical GmbH) angetrieben. Bewährt haben sich auch handgehaltene, elektrisch angetriebene Lavage-Systeme. Es muss jedoch immer ein großer Batterieblock oder Akkumulatorenblock mitgeführt werden, der naturgemäß nur eine begrenzte Ladungskapazität hat. Batterie- und Akkumulatorenblöcke sind im Hinblick auf ihre Umweltfreundlichkeit kritisch zu sehen. Druckgasgetriebene Lavage-Systeme haben den Vorteil, dass Druckluft im Operationssaal meist unlimitiert zur Verfügung steht und damit beliebig lange Spülflüssigkeit versprüht werden kann, ohne dass die Energiezufuhr begrenzt ist.

Bei den mit Druckluft oder einem anderen komprimierten Gas getriebenen Systemen wird üblicherweise ein Druckgasmotor zum Antrieb eingesetzt. Bei den meisten Druckgasmotoren für Lavage-Systeme handelt es sich um Lamellen-Druckgasmotoren. Der Druckgasmotor erzeugt eine Drehbewegung, die dann in eine oszillierende, lineare Bewegung umgesetzt wird. Die oszillierende lineare Bewegung wird genutzt, um jeweils kleinen Volumina des Spülmediums einen Impuls zu verleihen. Dabei wird üblicherweise mindestens eine Membran zwischen dem Antrieb und dem Zulauf der Spülflüssigkeit angeordnet, um die Impulse auf die Spülflüssigkeit übertragen zu können. Es entstehen dadurch Sprühstöße mit hohen Pulszahlen von 2000 bis 3000 Impulsen pro Minute. Das bedeutet, dass der Druckgasmotor sehr präzise gefertigt sein muss, um entsprechend hohe Drehzahlen zu tolerieren. Weiterhin muss eine entsprechend stabile Lagerung vorhanden sein. Aus diesen Gründen ist der Druckgasmotor bei üblichen Druckgas-getriebenen Lavage-Systemen das kostenintensivste Bauteil. Es wird deshalb im Allgemeinen der Druckgasmotor in einem Handgriff aus Metall oder anderen langzeitstabilen Materialien angeordnet, so dass dieses Bauteil mehrfach nach entsprechender Aufbereitung und Sterilisation genutzt werden kann.

Ein grundsätzliches Problem der Lamellen-Druckgasmotoren besteht darin, dass zu ihrem Betrieb sehr große Gasvolumina benötigt werden. Der Wirkungsgrad der Lamellen-Druckgasmotoren ist relativ niedrig. Wünschenswert wäre jedoch ein kostengünstiger Druckgasmotor, der mit geringen Gasvolumina betrieben werden kann, so dass unabhängig von einer stationären Druckluftversorgung, durch Verwendung von kleinvolumigen Gaspatronen, bevorzugt von Gaspatronen mit verflüssigtem Gas, Lavage-Systeme ortsunabhängig, ohne externe Energiequelle, angetrieben werden könnten. Bei Verwendung eines kostengünstigen Druckgasmotors, der aus preisgünstigen Kunststoffen gefertigt ist, wäre es möglich, durch Druckgas angetriebene Lavage-Systeme herzustellen, die nur zum einmaligen Gebrauch bestimmt sind und nach Verwendung kostengünstig entsorgt werden können. Dies ist auch aus Hygiene-Gründen bei medizinischen Operationen besonders vorteilhaft.

Druckgasmotoren, die Kolben enthalten, sind seit langem allgemein bekannt. Zum Betrieb dieser Druckgasmotoren muss der Zustrom des Druckgases durch Einlassventile gesteuert werden und nach Expansion des Gases muss das Ausströmen des entspannten Gases durch Auslassventile erfolgen. Das bedeutet, dass das Gaseinlassventil und das Gasauslassventil exakt zeitlich abgestimmt werden müssen und je nach Kolbenposition geöffnet und geschlossen werden. Für die Funktion ist dann eine präzise zeitliche Ventilsteuerung wesentlich. Die Ventilsteuerung kann dabei nach verschiedenen Prinzipien erfolgen. Häufig erfolgt eine Ventilsteuerung über Nocken, die an einer Nockenwelle angeordnet sind. Problematisch ist bei Druckgasmotoren immer, dass ein sogenannter "toter Punkt" überwunden werden muss, an dem der Druckgasmotor stillstehen kann, ohne dass er durch das Druckgas wieder in Gang gesetzt werden kann. Dies wird vielfach durch geeignete rotierende Schwungmassen erreicht. Die bisher in der Technik üblichen Druckgasmotoren, die Kolben nutzen, sind sehr komplex aufgebaut und benötigen eine exakte zeitliche Ventilsteuerung. Diese Druckgasmotoren sind für einmalig zu verwendende Lavage-Systeme auf Grund ihrer Komplexität und ihrer hohe Kosten nicht geeignet.

Ein gattungsgemäßer Druckgasmotor ist aus der WO 2012/038003 A1 bekannt. Der dort beschriebene Druckgasmotor hat einen zweiteiligen Kolben mit einem Zwischenraum und einen Durchgang durch einen der Kolben. Dadurch ist der Motor besonders einfach und kostengünstig im Aufbau.

Es besteht immer der Wunsch nach einem kostengünstiger aufzubauenden Druckgasmotor. Zudem besteht auch ein Bedürfnis danach, einen Druckgasmotor bereitzustellen, der mit einer höheren Frequenz und/oder einer größeren Kraft betrieben werden kann.

Solche Druckgasmotoren sind aber nicht nur für Lavage-Systeme geeignet, sondern können auch überall dort eingesetzt werden, wo ein Druckgas und insbesondere Druckluft zur Verfügung steht und ein kostengünstiger Antrieb vorteilhaft ist. Solche Voraussetzungen sind beispielsweise bei Rüttelanlagen gegeben, bei denen ein Schüttgut oder Pulver transportiert, abgefüllt beziehungsweise portioniert werden muss. Ebenso können solche Druckgasmotoren als Pumpen zur Bereitstellung von Schmiermitteln vorteilhaft eingesetzt werden. Ferner wäre auch die Verwendung in kostengünstig zu fertigenden Spielzeugen vorteilhaft.

Die Aufgabe der Erfindung besteht also darin, die Nachteile des Stands der Technik zu überwinden. Insbesondere soll ein kostengünstiger und zuverlässiger Druckgasmotor gefunden werden, der für die genannten Zwecke einsetzbar ist. Eine Aufgabe der Erfindung ist dabei darin zu sehen, einen maximal vereinfachten Kolben-Druckgasmotor zu entwickeln, der eine periodische, lineare Kolbenbewegung erzeugen kann. Dabei ist es wichtig, dass der Druckgasmotor ohne komplexe, kostenintensive Ventilsysteme auskommt und so vereinfacht wird, dass die Bauteile des Druckgasmotors kostengünstig durch Kunststoffspritzgießen hergestellt werden können. Es müssen Ventilsysteme vermieden werden, die ein großes Volumen einnehmen und die separat vom Druckgasmotor positioniert werden müssen. In dem Druckgasmotor sollen daher die erforderlichen Ventilfunktionen möglichst platzsparend integriert sein, um eine Verwendung des Druckgasmotors als Antrieb in Handstücken von Lavage-Systemen zu ermöglichen. Die zeitliche Steuerung der Ventilfunktionen sollte idealerweise so sein, dass bei jedem Punkt der Kolbenbewegung ein "toter Punkt" vermieden wird. Weiterhin soll mit dem Druckgasmotor eine vereinfachte Pumpe entwickelt werden, die in handgehaltenen Lavage-Systemen untergebracht werden kann. Eine mit dem Druckgasmotor angetriebene Pumpe soll so vereinfacht sein, das diese derart kostengünstig hergestellt werden kann, dass eine Verwendung in Lavage-Systemen möglich ist, die zur nur einmaligen Verwendung bestimmt sind.

Die Aufgaben der Erfindung werden gelöst durch Druckgasmotor aufweisend einen Arbeitskolben, einen Innenraum, der an einer Rückseite geschlossen ist und in dem der Arbeitskolben linear beweglich angeordnet ist, ein Rückstellelement, das zumindest zeitweise eine in Richtung der Rückseite wirkende Kraft auf den Arbeitskolben ausübt, eine Gaseinlassöffnung zum Einspeisen eines unter Druck stehenden Gases in den Innenraum und eine Gasauslassöffnung zum Abführen des Gases aus dem Innenraum, der dadurch gekennzeichnet ist, dass zwischen dem Arbeitskolben und der geschlossenen Rückseite ein Steuerkolben linear beweglich im Innenraum angeordnet ist, wobei der Steuerkolben in einer ersten Position die Gasauslassöffnung abdeckt und die Gaseinlassöffnung nicht abdeckt und in einer zweiten Position die Gaseinlassöffnung abdeckt und die Gasauslassöffnung nicht abdeckt, der Steuerkolben gegen den Arbeitskolben beweglich gelagert ist und an dem Arbeitskolben und/oder dem Steuerkolben ein Mitnehmerelement angeordnet ist, das bei einer Bewegung des Arbeitskolbens vom Steuerkolben weg den Steuerkolben in Richtung des Arbeitskolbens mit einem ersten Abstand nachzieht.

Bei einem entsprechend vertikal aufgestellten Druckgasmotor kann sogar auf ein körperliches Rückstellelement verzichtet werden, da die Rückstellung des Arbeitskolbens dann durch die Schwerkraft erfolgen kann. Es kann also erfindungsgemäß vorgesehen sein, dass das Rückstellelement gebildet wird durch die vertikale Aufstellung des Druckgasmotors, durch die der Arbeitskolben mit Hilfe der Schwerkraft zur Rückseite beschleunigt wird. Der Druckgasmotor ist dann besonders einfach aufgebaut. Um den Druckgasmotor bezogen auf die Erdoberfläche variabel positionieren zu können, wie dies beispielsweise bei handgehaltenen Lavage-Systemen erforderlich ist, kann jedoch erfindungsgemäß bevorzugt vorgesehen sein, dass das Rückstellelement ein körperliches Rückstellelement ist, insbesondere eine elastische Feder, wie zum Beispiel eine Stahlfeder und/oder eine Gasfederung ist. Die elastische Feder kann sowohl eine Zugfeder als auch eine Druckfeder sein. Als Druckfeder ist die elastische Feder zwischen dem Arbeitskolben und einem, der Rückseite gegenüberliegenden vorderen Ende des Innenraums in dem Innenraum angeordnet. Als Zugfeder ist die elastische Feder zwischen der Rückseite des Innenraums und dem Arbeitszylinder im Innenraum angeordnet. Dazu muss die Zugfeder sowohl an der Rückseite des Innenraums als auch an dem Arbeitszylinder verankert sein. Bevorzugt ist die Zugfeder dabei an dem Mitnehmerelement verankert, wobei das Mitnehmerelement fest mit dem Arbeitszylinder verbunden ist. Besonders bevorzugt ist das Mitnehmerelement über eine Stange oder einen Mitnehmerstift mit dem Arbeitszylinder verbunden. Ganz besonders bevorzugt sind der Mitnehmerstift, beziehungsweise die Stange, der Mitnehmer und der Arbeitskolben einteilig ausgeführt. Als Rückstellelement kann alternativ oder zusätzlich auch eine Gasfeder verwendet werden, beispielsweise dadurch, dass der Innenraum zwischen dem Arbeitskolben und dem vorderen Ende des Innenraums nach außen gasdicht und druckdicht geschlossen ist.

Mit der Erfindung wird vorgeschlagen, dass der Innenraum zumindest bereichsweise zylindrisch ist oder im Bereich eines Arbeitsraums des Arbeitskolbens oder im gesamten Hubraum des Arbeitskolbens und des Steuerkolbens zylindrisch ist.

Der zylindrische Innenraum muss nicht vollständig zylindrisch zu sein. Es reicht aus, wenn der Hubraum des Arbeitszylinders zylindrisch ist. Bevorzugt ist auch der Hubraum des Steuerzylinders zylindrisch. Der Hubraum des Steuerzylinders muss nicht zylindrisch sein, wenn darin Fräsungen oder andere Ausnehmungen vorgesehen sind, die dazu dienen, dass das Druckgas am Steuerkolben vorbei in den Raum zwischen dem Steuerkolben und der Rückseite des Innenraums gelangen kann. Der Raum zwischen der maximalen Auslenkung des Arbeitszylinders in Richtung des vorderen Endes des Innenraums und dem vorderen Ende des Innenraums, in dem bevorzugt das Rückstellelement (besonders bevorzugt als elastische Feder ausgeführt) angeordnet ist, muss nicht zylindrisch sein. Der Aufbau des Druckgasmotors wird jedoch vereinfacht, wenn auch dieser Teil des Innenraums zylindrisch ist. Ein Zylinder im Sinne der vorliegenden Erfindung ist, wie auch nach der allgemeinen Definition, ein von zwei parallelen, ebenen, kongruenten Flächen (Grund- und Deckfläche) und einer Mantelfläche beziehungsweise Zylinderfläche begrenzter Körper, wobei die Mantelfläche von parallelen Geraden gebildet wird. Das heißt, der Zylinder entsteht durch Verschiebung einer ebenen Fläche oder Kurve entlang einer Geraden, die nicht in dieser Ebene liegt. Die Höhe des Zylinders ist gegeben durch den Abstand der beiden Ebenen, in denen Grund- und Deckfläche liegen.

Sind die Geraden senkrecht zu Grund- und Deckfläche, spricht man von einem geraden Zylinder. Die gerade zylindrische Geometrie des Innenraums ist erfindungsgemäß bevorzugt, bezieht sich aber meist nur auf einen Teilbereich des gesamten Innenraums. Ein gerader Kreiszylinder stellt im Sinne der vorliegenden Erfindung also nur einen Spezialfall einer zylindrischen Geometrie dar.

Der Arbeitskolben schließt in allen Positionen mit den Innenwänden des Innenraums dicht ab, so dass der Innenraum stets durch den Arbeitskolben in zwei voneinander getrennte Kammern getrennt ist. Die Trennung erfolgt bevorzugt gasdicht und druckdicht.

Erfindungsgemäß kann vorgesehen sein, dass der Steuerkolben in axialer Richtung des Innenraums mindestens so lang ist wie die Summe des axialen Abstands der Gaseinlassöffnung von der Gasauslassöffnung und der axialen Querschnitte der Gaseinlassöffnung und der Gasauslassöffnung. Die axialen Querschnitte der Gaseinlassöffnung und der Gasauslassöffnung sind die Querschnitte der Gaseinlassöffnung und der Gasauslassöffnung in axialer Richtung des insbesondere zylindrischen Innenraums.

Bevorzugt erfolgt die lineare Bewegung des Arbeitskolbens und des Steuerkolbens entlang einer Geraden, die der Symmetrieachse des zylindrischen Innenraums entspricht.

Bei einem erfindungsgemäßen Druckgasmotor kann erfindungsgemäß vorgesehen sein, dass der Steuerkolben in einer dritten Position zwischen der ersten Position und der zweiten Position sowohl die Gaseinlassöffnung als auch die Gasauslassöffnung abdeckt, insbesondere vollständig abdeckt.

Hierdurch wird sichergestellt, dass der Druckgasmotor nicht in einer Zwischenposition, in der die Gaseinlassöffnung und die Gasauslassöffnung gleichzeitig offen sind, stehenbleibt und durch einen solchen "Kurzschluss" stehenbleiben kann. Die Trägheit des Arbeitskolbens und/oder die Kraft des Rückstellelements sorgen dafür, dass diese beiden Öffnungen nicht dauerhaft verschlossen bleiben.

Des Weiteren kann vorgesehen sein, dass an der dem Arbeitskolben zugewandten Seite des Steuerkolbens zumindest ein Abstandhalter angeordnet ist und/oder an der dem Steuerkolben zugewandten Seite des Arbeitskolbens zumindest ein Abstandhalter angeordnet ist, so dass zwischen dem Arbeitskolben und dem Steuerkolben ein Zwischenraum entsteht, wenn diese aneinander anliegen, und die Gaseinlassöffnung in der ersten Position in diesen Zwischenraum mündet, wobei durch den zumindest einen Abstandhalter bei aneinander anliegenden Arbeitskolben und Steuerkolben ein zweiter Abstand zwischen dem Arbeitskolben und dem Steuerkolben eingestellt wird, der kleiner ist als der erste Abstand, der durch das Mitnehmerelement eingestellt wird.

Der Abstandshalter kann durch ein Rohr entlang der Mittelachse des Innenraums gebildet sein, durch das sich der Mitnehmer erstreckt, beziehungsweise der Mitnehmerstift oder die Stange erstreckt, an dem oder an der der Mitnehmer am Arbeitskolben oder am Steuerkolben befestigt ist. Alternativ kann der zumindest eine Abstandshalter auch als Stift ausgebildet sein. Bevorzugt ist der Abstandhalter oder sind die Abstandhalter einteilig mit dem Arbeitskolben oder dem Steuerkolben ausgebildet. Durch den oder die Abstandhalter wird sichergestellt, dass der Druckgasmotor in jedem Fall zum Laufen gebracht werden kann, indem das unter Druck stehende Gas zwischen den Arbeitskolben und den Steuerkolben eingeleitet werden kann.

Es ist für die Erfindung wesentlich, dass sich der Abstand zwischen dem Arbeitskolben und dem Steuerkolben vergrößert, wenn der Arbeitskolben durch das Druckgas aus der Ausgangsposition heraus in Richtung von der Rückseite weg beschleunigt wird. Dadurch wird erreicht, dass über das Mitnehmerelement ein Stoß oder eine größere Kraft zur Beschleunigung des Steuerkolbens aufgebracht werden kann, so dass der Druckgasmotor nicht durch einen ruhenden oder blockierenden Steuerkolben stillstehen kann. Zu dem gleichen Zweck kann erfindungsgemäß auch vorgesehen sein, dass der Arbeitskolben, und insbesondere der Arbeitskolben mit dem fest daran verbundenen Mitnehmerelement, eine größere Masse aufweist als der Steuerkolben.

Ferner wird mit der Erfindung vorgeschlagen, dass das Rückstellelement eine elastische Feder ist, die im Innenraum zwischen dem Arbeitskolben und einem vorderen Ende des Innenraums angeordnet ist, wobei das vordere Ende des Innenraums gegenüber der Rückseite des Innenraums angeordnet ist.

Hierdurch wird ein besonders einfach und kostengünstig aufzubauender Druckgasmotor bereitgestellt, der unabhängig von seiner Orientierung gut funktioniert.

Es kann auch vorgesehen sein, dass der Steuerkolben in der ersten Position von dem Rückstellelement über den Arbeitskolben an die Rückseite gedrückt oder gezogen ist, wobei die Gaseinlassöffnung in eine Öffnung oder den Zwischenraum zwischen dem Arbeitskolben und dem Steuerkolben mündet.

Durch diesen Aufbau gelingt ist, dass der Druckgasmotor in einer Situation, in der kein Druckgas durch die Gaseinlassöffnung in den Innenraum geleitet wird, in eine Startposition (die Ausgangsposition des Arbeitskolbens und des Steuerkolbens) gebracht wird, aus der er bei Einleiten eines Druckgases durch die Gaseinlassöffnung startet.

Gemäß einer bevorzugten Ausgestaltung der Erfindung kann auch vorgesehen sein, dass in dem Steuerkolben wenigstens ein gasdurchlässiger Durchgang angeordnet ist, die die dem Arbeitskolben zugewandte Vorderseite des Steuerkolbens mit der der Rückseite des Innenraums zugewandten Rückseite des Steuerkolbens verbindet. Die Verbindung ist eine gasdurchlässige Verbindung.

Der Steuerkolben kann dann vollumfänglich an den Innenwänden des Innenraums anliegen. Dadurch wird eine stabile lineare Führung des Steuerkolbens im Innenraum gewährleistet.

Bevorzugte Ausführungsformen der Erfindung können sich dadurch auszeichnen, dass der Arbeitskolben vollumfänglich an der Innenwand des Innenraums anliegt, bevorzugt über ein Dichtungselement vollumfänglich, gasdicht und druckdicht an dem Innenraum anliegt.

Hierdurch wird gewährleistet, dass das komprimierte Gas nicht an dem Arbeitskolben vorbei strömt und dadurch die Funktionstüchtigkeit oder Leistung des Druckgasmotors gemindert wird.

Für erfindungsgemäße Druckgasmotoren zur Erzeugung einer mechanischen Bewegung kann bevorzugt vorgesehen sein, dass an der dem Steuerkolben abgewandten Seite des Arbeitskolbens eine Stange, insbesondere eine Pleuelstange befestigt ist, die aus dem Innenraum herausragt, bevorzugt durch die Vorderseite des Innenraums herausragt.

Bei dieser Variante wird durch die Stange ein periodischer Stoß erzeugt. Bei Verwendung einer Pleuelstange, die über eine Achse mit dem Arbeitskolben verbunden ist, kann die periodische Bewegung des Arbeitskolbens in eine Drehbewegung umgesetzt werden. Dazu ist die Pleuelstange bevorzugt mit einer Kurbelwelle verbunden oder verbindbar.

Bei dieser Variante kann auch vorgesehen sein, dass die Stange durch eine gasdichte Führung durch die Vorderseite des Innenraums aus dem Innenraum geführt wird und dass der Innenraum zwischen der Vorderseite und dem Arbeitskolben gasdicht und druckdicht geschlossen ist und eine Gasfederung als Rückstellelement bildet.

Hierdurch kann auf ein separates Rückstellelement verzichtet werden oder die Gasfederung kann als zusätzliches Rückstellelement verwendet werden. Im ersten Fall wird der Druckgasmotor sehr kostengünstig aufzubauen sein im zweiten Fall kann eine stärkere Rückstellkraft erreicht werden und der Motor kann auch den Ausfall oder die Verschlechterung eines der Rückstellelemente verkraften.

Bei Ausführungen, die stattdessen unmittelbar zum Ausstoß einer Flüssigkeit vorgesehen sind, kann vorgesehen sein, dass in der der Rückseite gegenüberliegenden Vorderseite des Innenraums eine Ausstoßöffnung vorgesehen ist und in der Vorderseite und/oder in der seitlichen Wandung des Innenraums eine Flüssigkeitszufuhröffnung angeordnet ist, die zumindest zeitweise nicht vom Arbeitskolben abgedeckt ist und die im nicht abgedeckten Zustand zwischen dem Arbeitskolben und der Vorderseite des Innenraums angeordnet ist.

Durch diesen Aufbau wird der Hubraum des Arbeitskolbens zumindest teilweise zur Flüssigkeitsaufnahme beziehungsweise zur Aufnahme eines Gemischs aus Gas und Flüssigkeit genutzt. Wenn der Arbeitskolben in Richtung der Vorderseite des Innenraums bewegt wird, wird die Flüssigkeit, beziehungsweise das Flüssigkeits-Gas-Gemisch durch die Ausstoßöffnung ausgestoßen. Der Druckgasmotor ist dann unmittelbar für Lavage-Systeme einsetzbar.

Bei dieser Ausführungsform kann vorgesehen sein, dass die Ausstoßöffnung durch ein Ventilelement, insbesondere ein Lippenventil mit der Umgebung verbunden ist, wobei das Ventilelement bei einem ausreichenden Überdruck gegenüber dem Umgebungsdruck geöffnet und ansonsten geschlossen ist, und dass an der Flüssigkeitszufuhröffnung ein Rohr oder eine Schlauch mit einem Rückschlagventil angeschlossen ist, das sich bei einem Unterdruck im Innenraum zwischen dem Arbeitskolben und der Vorderseite des Innenraums öffnet und eine Flüssigkeitszufuhr in den Innenraum ermöglicht.

Hierdurch wird eine automatische Befüllung des Innenraums mit einer Flüssigkeit erreicht. Dadurch wird ein besonders einfacher und kostengünstiger Aufbau eines Druckgasmotors für ein Lavage-System erreicht. Zur Definition eines ausreichenden Drucks sei auf bekannte Lippenventile zu ähnlichen oder gleichen Zwecken verwiesen.

Erfindungsgemäße Druckgasmotoren können sich auch dadurch auszeichnen, dass vorgesehen ist, dass das Mitnehmerelement eine Schnur, ein Kabel, ein Faden, eine Kette oder eine elastische Feder ist, die am Arbeitskolben und am Steuerkolben befestigt ist oder das Mitnehmerelement eine Stange, eine Schnur, ein Kabel, ein Faden, eine Kette oder eine elastische Feder aufweist, die am Arbeitskolben oder am Steuerkolben befestigt ist und an der ein Mitnehmer befestigt ist, der in einen Vorsprung im Arbeitskolben oder im Steuerkolben greift, wobei bevorzugt das Mitnehmerelement durch eine am Arbeitskolben befestigte Stange gebildet wird, die durch eine Durchführung im Steuerkolben reicht und an der ein Mitnehmer befestigt ist, der nicht durch die Durchführung im Steuerkolben passt und der auf der Rückseite des Steuerkolbens in diesen eingreift um den Steuerkolben mitzuziehen, wenn der Arbeitskolben dazu ausreichend weit von dem Steuerkolben entfernt ist und sich in Richtung von diesem weg bewegt.

Mit den genannten Mitnehmerelementen kann auf baulich einfache Weise eine Vergrößerung des Abstands zwischen dem Arbeitskolben und dem Steuerkolben erreicht werden, bevor der Steuerkolben von dem Arbeitskolben mitgezogen wird. Dadurch kann der Wirkungsgrad des Motors verbessert und die Gefahr eines Stillstands des Motors verringert werden.

Ferner kann erfindungsgemäß bevorzugt vorgesehen sein, dass der Arbeitskolben zwei unterschiedlich große Querschnittsflächen senkrecht zur Bewegungsrichtung des Arbeitskolbens aufweist, wobei der Innenraum dazu passende Innenwände mit unterschiedlichen Querschnittsflächen aufweist und die Querschnittsfläche auf der, der Rückseite zugewandten Seite des Arbeitskolbens kleiner ist als die Querschnittsfläche der gegenüberliegenden Vorderseite des Arbeitskolbens. Bevorzugt kann dabei vorgesehen sein, dass die Querschnittsfläche auf der, der Rückseite zugewandten Seite des Arbeitskolbens höchstens halb so groß wie die Querschnittsfläche der gegenüberliegenden Vorderseite des Arbeitskolbens ist.

Dies hat den Vorteil, dass das verbrauchte Gasvolumen durch die Verkleinerung des Arbeitsraums, das heißt des Raums, in dem an dem Arbeitskolben die Arbeit durch das komprimierte Gas geleistet wird, geringer ist, als wenn beide Teile des Arbeitskolbens gleich groß sind. Hierdurch wird der Gasverbrauch des Druckgasmotors reduziert. Pro Arbeitszyklus muss dann also eine geringere Menge Druckgas durch den Druckgasmotor geleitet werden. Besonders bevorzugt ist bei dieser Variante vorgesehen, dass der Steuerkolben die gleiche geringe Querschnittsfläche aufweist, wie die der Rückseite zugewandte Seite des Arbeitskolbens. Ebenfalls ist es bei dieser Ausführungsform bevorzugt, dass die der Rückseite zugewandte Seite des Arbeitskolbens mit dem geringen Durchmesser bezüglich der Länge in Richtung der Bewegungsrichtung des Arbeitskolbens länger ist, als die der Vorderseite zugewandten Seite des Arbeitskolbens, besonders bevorzugt zumindest doppelt so lange ist, als die der Vorderseite zugewandten Seite des Arbeitskolbens. Ferner kann bei dieser Ausführungsform vorgesehen sein, dass um beide Seiten des Arbeitskolbens für jede der beiden Querschnittsflächen jeweils zumindest eine vollumfängliche Dichtung um den Arbeitskolben herum angeordnet ist, die den Arbeitskolben gegen die Innenwände des Innenraums abdichtet.

Bei ovalen oder kreisförmigen Querschnittsflächen des Arbeitskolbens kann anstatt von einer Querschnittsfläche immer auch von einem Radius, Durchmesser oder Querschnitt gesprochen werden. Dementsprechend kann bei ovalen oder kreisförmigen Querschnittsflächen des Arbeitskolbens oder bei anderen Querschnittsflächen des Arbeitskolbens, auf die ein Querschnitt, Radius oder Durchmesser anwendbar ist, erfindungsgemäß vorgesehen sein, dass der Arbeitskolben zwei unterschiedlich große Durchmesser oder Querschnitte senkrecht zur Bewegungsrichtung des Arbeitskolbens aufweist, wobei der Innenraum dazu passende Innenwände mit unterschiedlichen Durchmessern oder Querschnitten aufweist und der Durchmesser oder der Querschnitt auf der, der Rückseite zugewandten Seite des Arbeitskolbens kleiner ist als der Durchmesser oder der Querschnitt der gegenüberliegenden Vorderseite des Arbeitskolbens. Bevorzugt kann dabei vorgesehen sein, dass der Durchmesser oder der Querschnitt auf der, der Rückseite zugewandten Seite des Arbeitskolbens höchstens halb so groß wie der Durchmesser oder der Querschnitt der gegenüberliegenden Vorderseite des Arbeitskolbens ist.

Die Aufgaben der Erfindung bezüglich eines Lavage-Systems werden gelöst durch ein Lavage-System aufweisend zumindest einen solchen Druckgasmotor, bei dem mit dem Druckgasmotor oder den Druckgasmotoren ein periodischer Sprühstoß mit einer Flüssigkeit erzeugbar ist.

Bei dem Lavage-System kann vorgesehen sein, dass das Lavage-System einen Anschluss für eine Druckgaspatrone, einen Druckminderer, einen Verdampfungsraum zum Verdampfen von flüssigen Flüssiggasresten und ein manuell betätigbares Steuerungsventil aufweist, wobei der Verdampfungsraum über eine Druckgasleitung mit dem Anschluss verbunden ist, der Druckminderer über eine Druckgasleitung mit dem Verdampfungsraum verbunden ist, das Steuerungsventil über eine Druckgasleitung mit dem Druckminderer verbunden ist und die Gaseinlassöffnung des Druckgasmotors über eine Druckgasleitung mit dem Steuerungsventil verbunden ist und wobei vorzugsweise eine Flüssigkeitszufuhröffnung des Druckgasmotors mit einer Flüssigkeitsleitung verbunden ist.

Die Aufgaben der Erfindung werden ferner gelöst durch die Verwendung eines solchen Druckgasmotors als Motor für ein Lavage-System, einen Klopfermotor, als Motor für ein Spielzeug, einen Vibrationsmotor, als Antrieb für eine Dosiervorrichtung, als Rüttelmotor oder als Pumpe, insbesondere als Schmiermittelpumpe.

Die der Erfindung zugrundeliegenden Aufgaben werden auch gelöst durch ein Verfahren zum Erzeugen einer periodischen Bewegung mit einem Druckgas, insbesondere unter Verwendung eines solchen erfindungsgemäßen Druckgasmotors bei dem
A) in einem Ausgangszustand der Arbeitskolben und der Steuerkolben in dem Innenraum voneinander mit dem ersten Abstand beabstandet werden, wobei der Steuerkolben die Gasauslassöffnung verschließt und die Gaseinlassöffnung zwischen dem Arbeitskolben und dem Steuerkolben offen liegt;
B) das Druckgas zwischen den Arbeitskolben und den Steuerkolben in den Innenraum geleitet wird;
C) der Arbeitskolben durch den Druck des Druckgases in Richtung einer Vorderseite des Innenraums vom Steuerkolben weg beschleunigt und bewegt wird, wobei sich der Abstand zwischen dem Arbeitskolben und dem Steuerkolben vergrößert;
D) der Steuerkolben über das Mitnehmerelement von dem Arbeitskolben mitgezogen wird, bevorzugt ab Erreichen des zweiten Abstands;
E) der Steuerkolben die Gaseinlassöffnung aufgrund der Bewegung des Steuerkolbens verschließt;
F) der Steuerkolben die Gasauslassöffnung aufgrund der Bewegung des Steuerkolbens öffnet;
G) der Arbeitskolben durch das Rückstellelement in Richtung der Rückseite des Innenraums beschleunigt wird;
H) das Druckgas zwischen dem Arbeitskolben und dem Steuerkolben durch wenigstens eine gasdurchlässige Öffnung durch den Steuerkolben in den Raum zwischen der Rückseite des Steuerkolbens und der Rückwand des Innenraums strömt;
I) das Druckgas in dem Raum zwischen der Rückseite des Steuerkolbens und der Rückwand des Innenraums durch die geöffnete Gasauslassöffnung an die Umgebung abgegeben wird;
J) der Arbeitskolben auf den Steuerkolben trifft und diesen in Richtung der Rückseite des Innenraums bewegt;
K) durch die umgekehrte Bewegung des Steuerkolbens die Gasauslassöffnung wieder geschlossen wird und
L) durch die umgekehrte Bewegung des Steuerkolbens die Gaseinlassöffnung wieder geöffnet wird, so dass erneut Druckgas zwischen den Arbeitskolben und den Steuerkolben in den Innenraum geleitet wird.

Die Bewegung des Arbeitskolbens in Schritt J) erfolgt aufgrund der Beschleunigung durch das Rückstellelement. Das Druckgas wirkt dagegen beim Auftreffen des Arbeitskolbens auf den Steuerkolben keine Kraft mehr auf den Arbeitskolben aus, da der Druck beziehungsweise das Druckgas bereits durch die geöffnete Gasauslassöffnung entwichen ist.

Die Schritte finden in der logischen teilweise chronologischen Reihenfolge statt, wobei sich die Zeiträume, zu denen die erfindungsgemäßen Schritte stattfinden, teilweise und zeitweise überlagern. Bevorzugt wird der Motor durch das Rückstellelement wieder in den Ausgangszustand zurückgeführt. Der Druckgasmotor funktioniert jedoch auch, wenn der Ausgangszustand erst wieder erreicht wird, wenn kein Druckgas mehr an der Gaseinlassöffnung zur Verfügung steht.

Das Druckgas wird mit einem Druck von wenigstens 150 kPa eingeleitet, bevorzugt mit einem Druck von wenigstens 300 kPa. Als Druckgasquelle wird erfindungsgemäß bevorzugt eine CO₂-Patrone mit flüssigem CO₂ verwendet. Der Druck aus der CO₂-Patrone wird erfindungsgemäß besonders bevorzugt mit Hilfe eines Druckminderers (Druckminderungsventils) auf einen Wert zwischen 150 kPa und 500 kPa reduziert, bevor er durch die Gaseinlassöffnung in den Druckgasmotor eingespeist wird.

Bei erfindungsgemäßen Verfahren kann vorgesehen sein, dass der Zyklus sich mit dem erneuten Einspeisen des Druckgases wiederholt.

Ferner kann erfindungsgemäß vorgesehen sein, dass der erste Abstand zwischen dem Arbeitskolben und dem Steuerkolben mit Hilfe zumindest eines Abstandhalters eingestellt wird und der zweite Abstand zwischen dem Arbeitskolben und dem Steuerkolben mit einem Mitnehmerelement eingestellt wird, vorzugsweise durch die Länge des Mitnehmerelements.

Bevorzugt wird der zweite Abstand zumindest doppelt so groß wie der erste Abstand gewählt.

Die der Erfindung zugrundeliegenden Aufgaben werden auch gelöst durch ein Verfahren zum Erzeugen eines Sprühstoßes aufweisend die genannten erfindungsgemäßen Verfahrensschritte, wobei bei einer Bewegung des Arbeitskolbens weg von der Rückseite des Innenraums eine Flüssigkeit oder ein Flüssigkeit-Gas-Gemisch aus dem Raum zwischen dem Arbeitskolben und der Vorderseite des Innenraums durch eine Ausstoßöffnung an der Vorderseite des Innenraums hinaus gestoßen wird und
bei einer Bewegung des Arbeitskolbens auf die Rückseite des Innenraums zu eine Flüssigkeit oder ein Flüssigkeit-Gas-Gemisch in den Raum zwischen dem Arbeitskolben und der Vorderseite des Innenraums durch eine Flüssigkeitszufuhröffnung hinein gedrückt oder gezogen wird.

Bei diesem Verfahren kann vorgesehen sein, dass bei der Bewegung des Arbeitskolbens auf die Vorderseite des Innenraums zu durch den Druck in dem Raum zwischen dem Arbeitskolben und der Vorderseite des Innenraums ein Ventil an der Ausstoßöffnung geöffnet wird und/oder offen gehalten wird und ein an die Flüssigkeitszufuhröffnung angeschlossenes Rückschlagventil geschlossen wird und/oder geschlossen gehalten wird und bei der Bewegung des Arbeitskolbens auf die Rückseite des Innenraums zu durch den geringeren Druck in dem Raum zwischen dem Arbeitskolben und der Vorderseite des Innenraums das Ventil an der Ausstoßöffnung geschlossen wird und/oder geschlossen gehalten wird und das an die Flüssigkeitszufuhröffnung angeschlossene Rückschlagventil geöffnet wird und/oder offen gehalten wird.

Der Erfindung liegt die überraschende Erkenntnis zugrunde, dass es durch die im Abstand zueinander veränderlichen Arbeits- und Steuerkolben gelingt, einen einfachen aber gleichzeitig zuverlässig laufenden Druckgasmotor bereitzustellen, der keinen Totpunkt aufweist, an dem der Druckgasmotor zum Stehen kommt, ohne dass er wieder anlaufen würde. Der "tote Punkt" wird erfindungsgemäß dadurch vermieden, dass bei dem Druckgasmotor immer ein definierter Zustand der Ventile möglich ist. Unter definiertem Zustand ist zu verstehen, dass ein Ventil entweder geöffnet oder geschlossen ist. Durch eine geeignete Einstellung der beiden Abstände der beiden Kolben zueinander während des Betriebs des Druckgasmotors kann sichergestellt werden, dass der Druckgasmotor passend zu dem jeweiligen Zweck einsetzbar ist. Insbesondere als Pumpe zum Erzeugen von Sprühstößen einer Flüssigkeit kann der Druckgasmotor auf einfachste Weise ausgelegt werden, indem der Hubraum des Arbeitskolbens unmittelbar als Pumpe verwendet wird.

Der Arbeitskolben und der Steuerkolben bewegen sich dabei nicht gleichzeitig mit konstantem Abstand zueinander. Zunächst bewegt sich der Arbeitskolben. Wenn der Arbeitskolben eine gewisse Entfernung zum Steuerkolben und dadurch eine gewisse Geschwindigkeit erreicht hat, zieht dieser den Steuerkolben ruckartig nach, indem die Kabel, Schnüre oder Ketten des Mitnehmerelements plötzlich gespannt werden oder in dem der Mitnehmer auf den Vorsprung des Steuerkolbens oder des Arbeitskolbens schlägt und dadurch der Steuerkolben mitgerissen wird. Die der vorliegenden Erfindung zugrundeliegende Idee besteht also insbesondere darin, eine zeitliche Entkopplung der Ventilsteuerung durch den Steuerkolben und der Bewegung des Arbeitskolbens zu erreichen. Hierdurch kann auf überraschende Weise eine kraftvolle Bewegung des Arbeitskolbens erreicht werden. Auch im Vergleich zu dem Druckgasmotor nach der WO 2012/038003 A1 mit dem zweiteiligen aber fest verbundenen Arbeitskolben ergibt sich mit dem erfindungsgemäßen Druckgasmotor eine kräftiger Bewegung des Arbeitskolbens, bei der also mit jeder Bewegung des Arbeitskolbens mehr Kraft übertragen werden kann.

Dadurch ist es möglich, den vorderen Teil des Hohlraums unmittelbar und ohne ein Getriebe oder eine Übersetzung zur Kraftverstärkung als Pumpraum einzusetzen. Der Sprühstoß eines Lavage-Systems kann so unmittelbar mit Hilfe des Arbeitskolbens erzeugt werden. Dadurch lassen sich Energieverluste beim Pumpen vermeiden. Ein Defekt des Getriebes oder eines Teils der Übersetzung ist hierdurch ausgeschlossen. Zudem wird der Aufbau kompakter und kostengünstiger, als wenn solche Teile angebaut oder eingebaut werden müssten.

Die Erfindung basiert dabei auf folgender Idee. Ein axial verschiebbarer Arbeitskolben wird in einem Hohlzylinder durch ein Rückstellelement gegen einen Steuerkolben gedrückt. In der Mantelfläche des Hohlzylinders sind eine Gaseinlassöffnung und eine Gasauslassöffnung getrennt voneinander angeordnet, die gasdurchlässig mit dem Innenraum des Hohlzylinders verbunden sind. Die Gaseinlassöffnung befindet sich in Richtung des Arbeitskolbens und des Rückstellelements und die Gasauslassöffnung ist entgegengesetzt dazu in Richtung des gasundurchlässigen Verschlusses des Hohlzylinders angeordnet. Der Steuerkolben ist axial im Hohlzylinder verschiebbar und kann je nach Position entweder nur Gasauslassöffnung verschließen, wobei gleichzeitig die Gaseinlassöffnung geöffnet ist, oder nur die Gaseinlassöffnung verschließen wobei gleichzeitig die Gasauslassöffnung geöffnet ist. Der Steuerkolben besitzt einen Durchgang, der sich axial durch den Steuerkolben erstreckt.

An der Stirnseite des Arbeitskolbens, die dem Rückstellelement entgegengesetzt ist, befindet sich ein Mitnehmerstift, an dessen Ende ein Mitnehmer angeordnet ist. Der Mitnehmerstift ist in dem Hohlraum des Steuerkolbens axial beweglich. Am Steuerkolben befindet sich in Richtung der Stirnseite des Arbeitskolbens, an dem der Mitnehmerstift angeordnet ist, ein Anschlag. An diesem kann der Mitnehmer bei axialer Bewegung des Mitnehmerstifts infolge der Bewegung des Arbeitskolbens in Richtung des Rückstellelements ansetzen und bei der axialen Bewegung den Steuerkolben in Richtung des Rückstellelements bewegen. Zwischen dem Arbeitskolben und dem Steuerkolben befindet sich ein Hohlraum, der durch zumindest einen Abstandhalter gehalten wird, wenn der Arbeitskolben und der Steuerkolben über den Abstandhalter aneinander anliegen, und der den Arbeitskolben zum Steuerkolben beabstandet. Dieser Hohlraum befindet sich ohne Beaufschlagung mit Druckgas direkt über der Gaseinlassöffnung.

Ein erfindungsgemäßer Druckgasmotor kann dazu beispielsweise zusammengesetzt sein aus:
a) einem Hohlzylinder, der mindestens eine Gaseinlassöffnung und eine Gasauslassöffnung besitzt, die an der Mantelfläche des Hohlzylinder getrennt voneinander angeordnet sind,
b) einem im Hohlzylinder axial beweglich angeordneten Arbeitskolben,
c) einem im Hohlzylinder angeordneten elastischen Rückstellelement, das an eine vorderen Stirnseite des Arbeitskolbens angreift,
d) einem Mitnehmerstift der an der dem Rückstellelement entgegengesetzten Stirnseite des Arbeitskolbens mit dem Arbeitskolben verbunden ist,
e) einem Mitnehmer der mit dem Mitnehmerstift verbunden ist,
f) einem axial im Hohlzylinder verschiebbaren Steuerkolben, der in axialer Achse mindestens so lang ist wie die Summe des axialen Abstands zwischen der Gaseinlassöffnung und der Gasauslassöffnung und der axialen Länge der Gaseinlassöffnung und/oder der Gasauslassöffnung, wobei der Steuerkolben bei axialer Verschiebung über die Gaseinlassöffnung die Gaseinlassöffnung gasdicht verschließt und gleichzeitig die Gasauslassöffnung geöffnet ist, der Steuerkolben bei Verschiebung über die Gasaustrittsöffnung die Gasauslassöffnung gasdicht verschließt und gleichzeitig die Gaseinlassöffnung geöffnet ist, der Steuerkolben mindestens einen Hohlraum besitzt, in dem der Mitnehmerstift axial verschoben werden kann,
g) einem Anschlag am Steuerkolben, der an der dem Arbeitskolben zugewandten Seite des Steuerkolbens so angeordnet ist, dass bei Bewegung des Arbeitskolbens in Richtung des Rückstellelements der Mitnehmerstift durch den Hohlraum des Steuerkolbens bewegt wird und dann der am Mitnehmerstift angeordnete Mitnehmer auf den Anschlag trifft und den Steuerkolben bei weiterer Bewegung des Arbeitskolbens in Richtung Rückstellelement bewegt,
h) einem Anschlag der die axiale Bewegung des Steuerkolbens entgegengesetzt zum Rückstellelement begrenzt und
i) einem gasundurchlässigen Verschluss an dem dem Rückstellelement entgegengesetztem Ende des Holzylinders, so dass ein Hohlraum durch den Hohlzylinder gebildet wird, der durch den gasundurchlässigen Arbeitskolben und den gasdurchlässigen Steuerkolben abgegrenzt wird.

Der Druckgasmotor funktioniert in der Weise, dass zuerst Druckgas durch die im Ruhezustand offene Gaseinlassöffnung in den Hohlraum (Innenraum) gelangt. Das Druckgas treibt den Arbeitskolben in Richtung Rückstellelement. Dabei bewegt sich der Mitnehmerstift mit dem Mitnehmer innerhalb des Hohlraums des Steuerkolbens mit, ohne dass die Position des Steuerkolbens verändert wird. Nach weiterer axialer Bewegung des Arbeitskolbens in Richtung des Rückstellelements schlägt der Mitnehmer an den Anschlag des Steuerkolbens. Daraufhin wird der Steuerkolben in Richtung des Rückstellelements durch den Mitnehmer mitgenommen, wobei die Gaseinlassöffnung durch den verschobene Steuerkolben verschlossen wird und unmittelbar danach die Gasauslassöffnung geöffnet wird. Wesentlich ist dabei, dass der Steuerkolben eine solche axiale Ausdehnung hat, dass es geometrisch unmöglich ist, dass gleichzeitig sowohl die Gaseinlassöffnung als auch die Gasauslassöffnung geöffnet sind. Durch die Trägheit des bewegten Arbeitskolbens wird hierbei der sogenannte "tote Punkt" überfahren, ohne dass dazu Schwungräder oder ähnliche Vorrichtungen oder träge Massen notwendig sind. Nach Öffnung der Gasauslassöffnung entweicht das zumindest teilentspannte Gas in die umgebende Atmosphäre. Dadurch sinkt der Druck hinter dem Arbeitskolben. Sobald die Rückstellkraft des Rückstellelements größer ist als die auf die Arbeitskolbenfläche einwirkende Druckkraft des noch vorhandenen, restlichen, zumindest teilentspannten Gases, wird der Arbeitskolben vom Rückstellelement zurück in die Ausgangsposition bewegt, wobei durch den Arbeitskolben der Steuerkolben auch in die Ausgangslage zurück gedrückt wird. Der Mitnehmerstift bewegt sich axial im Hohlraum des Steuerkolbens ebenfalls mit zurück, wobei der Mitnehmer vom Anschlag gelöst wird und ebenfalls in seine Ausgangsposition zurückkehrt. Der Hohlraum zwischen dem Arbeitskolben und dem Steuerkolben befindet sich wieder oberhalb der Gaseinlassöffnung. Danach beginnt der beschriebene Prozess erneut und erfolgt so lange wie Druckgas an der Gaseinlassöffnung anliegt.

Der Arbeitskolben ist gasdicht axial im Hohlzylinder verschiebbar. Die Dichtung kann durch O-Ringe oder auch durch eine nicht zu feste Passung erreicht werden.

Der Steuerkolben ist innerhalb des Hohlzylinders axial verschiebbar, wobei je nach Position des Steuerkolbens im Hohlzylinder entweder die Gaseinlassöffnung oder die Gasauslassöffnung gasdicht geschlossen sind, wobei gleichzeitig entweder nur die Gasauslassöffnung oder nur die Gaseinlassöffnung geöffnet sind. Bevorzugt kann immer nur die Gaseinlassöffnung oder die Gasauslassöffnung geöffnet sein, um einen "Kurzschluss" bezogen auf die Strömung des Druckgases zu verhindern.

Es ist mindestens ein Abstandhalter am Arbeitskolben und/oder am Steuerkolben angeordnet, welcher den Steuerkolben vom Arbeitskolben beabstandet, wobei erfindungsgemäß bevorzugt der Mindestabstand zwischen dem Steuerkolben und dem Arbeitskolben zumindest 0,1 mm beträgt. Dieser Mindestabstand ist notwendig, damit in diesen Hohlraum (beziehungsweise Zwischenraum oder Spalt) das Druckgas eindringen kann und den Arbeitskolben unter Volumenvergrößerung des Hohlraums bewegt.

Wichtig ist, dass ohne Einwirkung des Druckgases, durch den Druck des Rückstellelements auf den Arbeitskolben, dieser derart positioniert ist, dass der durch den mindestens einen Abstandhalter gebildete Hohlraum im Hohlzylinder zwischen dem Steuerkolben und dem Arbeitskolben gasdurchlässig nur mit der Gaseinlassöffnung verbunden ist, wobei gleichzeitig die Gasauslassöffnung durch den Steuerkolben verschlossen ist. Das bedeutet, dass in der Ausgangslage des Druckgasmotors, ohne Beaufschlagung mit Druckgas, der Hohlraum immer genau über der Gaseinlassöffnung liegt, damit nach Beaufschlagung mit Druckgas der Hohlraum unter Bewegung des Arbeitskolbens vergrößert werden kann. Dadurch ist ein automatisches Starten des Druckgasmotors aus einer definierten Ausgangslage (der Startposition) des Arbeitskolbens und des Steuerkolbens gewährleistet.

Für die Funktion des Druckgasmotors ist es notwendig, dass der Steuerkolben mindestens einen axialen Gasdurchlass (beziehungsweise einen Durchgang) besitzt, der den durch den Hohlzylinder, der Stirnseite des Arbeitskolbens und dem Steuerkolben gebildeten Hohlraum gasdurchlässig mit dem von der entgegengesetzten Stirnseite des Steuerkolbens, dem Hohlzylinder und dem gasundurchlässigen Verschluss gebildeten Hohlraum verbindet. Dadurch kann nach Verschluss der Gaseinlassöffnung das unter Druck stehende Gas zur geöffneten Gasauslassöffnung gelangen und in die umgebende Atmosphäre austreten. Dadurch ist ein Druckabbau möglich, so dass bei dadurch sinkendem Gasdruck das Rückstellelement in der Lage ist, den Arbeitskolben und den Steuerkolben in die Ausgangsstellung zurück zu drücken, wobei die Gaseinlassöffnung geöffnet und die Gasauslassöffnung geschlossen wird. Der Antriebsprozess beginnt daher dann erneut. Durch periodische Wiederholung dieses Prozesses wird eine periodische lineare Bewegung des Arbeitskolbens erzeugt. Der Prozess läuft ohne Einwirkung äußerer Ventile selbstständig ab, solange eine Beaufschlagung mit Druckgas erfolgt.

Als Rückstellelement werden Spiralfedern und Blattfedern erfindungsgemäß besonders bevorzugt. Diese können im Rahmen der vorliegenden Erfindung aus Stahl, Edelstahl oder auch aus Kunststoffen gefertigt sein. Wesentlich ist, dass das Rückstellelement den Arbeitskolben und den Steuerkolben nach erfolgtem Gasauslass in die Ausgangsstellung zurück treiben kann.

Erfindungsgemäß kann ferner vorgesehen sein, dass der Druckgasmotor aus thermoplastischen Kunststoffen gefertigt ist, wobei Polypropylen, Polyethylen, Polyamid-6 und Polyamid-12 besonders bevorzugt sind. Daneben sind alle technisch üblichen Kunststoffe geeignet.

Ein erfindungsgemäßes Verfahren zur Erzeugung einer periodischen, linearen Bewegung mit einem erfindungsgemäßen Druckgasmotor kann beispielsweise dadurch realisiert werden, dass
a) zuerst durch das Rückstellelement der Arbeitskolben in Richtung des Anschlags (der Rückseite beziehungsweise Rückplatte) so geschoben ist, dass der Steuerkolben am Anschlag anliegt, wobei die Gaseinlassöffnung geöffnet ist und die Gasauslassöffnung durch Überdeckung des Steuerkolbens gasundurchlässig verschlossen ist,
b) durch die offene Gaseinlassöffnung Druckgas einströmt und den Arbeitskolben in Richtung des Rückstellelements bewegt, wobei der an der Rückseite des Arbeitskolbens angeordnete Mitnehmerstift ebenfalls in Richtung des Rückstellelements durch den axialen Hohlraum des Steuerkolbens mit bewegt wird, bis der Mitnehmer den Anschlag am Steuerkolben erreicht,
c) anschließend der Mitnehmer an den Anschlag des Steuerkolbens greift und bei der weiteren Bewegung des Arbeitskolbens in Richtung des Rückstellelements den Steuerkolben axial in Richtung des Rückstellelements verschiebt, wodurch die Gaseinlassöffnung durch den Steuerkolben verschlossen wird und die Gasauslassöffnung geöffnet wird,
d) daran anschließend das Druckgas durch die geöffnete Gasauslassöffnung aus den Hohlzylinder in die Umgebung strömt, bis der Gasdruck so niedrig ist, dass die Rückstellkraft des Rückstellelements größer ist als die Kraft des im Hohlzylinder verbliebenen restlichen Druckgases, wodurch das Rückstellelement den Arbeitskolben in Richtung des Steuerkolbens verschiebt, wodurch der Arbeitskolben über den mindestens einen Abstandhalter den Steuerkolben ebenfalls entgegengesetzt zum Rückstellelement axial verschiebt, wodurch die Gaseintrittsöffnung durch den Steuerkolben geöffnet wird und die Gasauslassöffnung verschlossen wird, und der Prozess dann wiederholt wird.

Der Prozess wird wiederholt, so lange das Druckgas über die Gaseinlassöffnung eingespeist wird.

Wesentlich für die Erfindung ist, dass die periodische, lineare Bewegung des Arbeitskolbens durch den Druckgasmotor selbständig durch Einwirkung von Druckgas ohne Einwirkung äußerer Ventile ausgelöst wird.

Für die Erzeugung von periodischen, linearen Bewegungen mit dem Druckgasmotor ist ein Druckgas notwendig, dass einen Druck von größer, gleich 0,5 bar (50 kPa) gegenüber der umgebenden Atmosphäre hat.

Erfindungsgemäß kann des Weiteren vorgesehen sein, dass als Druckgas bevorzugt Kohlendioxid, Luft, Stickstoff, Distickstoffmonoxid oder deren Gemische verwendet werden. Besonders vorteilhaft ist dabei die Verwendung von flüssigem Kohlendioxid, das kostengünstig kommerziell in Metallpatronen verfügbar ist. Durch die Verwendung von flüssigem Kohlendioxid können große Gasvolumina in relativ kleinen Metallpatronen platzsparend untergebracht werden. Dadurch ist eine Unterbringung dieser Patronen in Handstücken von Lavage-Systemen (beziehungsweise handgehaltenen Lavage-Systemen) möglich. Daneben sind auch andere Druckgase verwendbar, einschließlich von Wasserdampf und von Gemischen aus Wasserdampf, Kohlendioxid, Kohlenmonoxid.

Ein weiteres Ausführungsbeispiel für die Erfindung ist eine druckgasgetriebene Flüssigkeitspumpe mit einem erfindungsgemäßen Druckgasmotor zusammengesetzt aus
a) einem Hohlzylinder, der mindestens eine Gaseinlassöffnung und eine Gasauslassöffnung besitzt, die an der Mantelfläche des Hohlzylinder getrennt voneinander angeordnet sind,
b) einem im Hohlzylinder axial beweglich angeordneten Arbeitskolben,
c) einem Hohlraum der durch die Mantelfläche des Hohlzylinders, der Stirnseite des Arbeitskolbens und der flüssigkeitsdichten Stirnfläche des Hohlzylinders gebildet wird, wobei dieser Hohlraum flüssigkeitsdurchlässig mit einem Rückschlagventil verbunden ist, wobei der Hohlraum flüssigkeitsdurchlässig mit einem Auslassventil verbunden ist,
d) einem im Hohlzylinder angeordneten elastischen Rückstellelement, das an eine Stirnseite des Arbeitskolbens angreift, und sich an der flüssigkeitsdichten Stirnfläche des Hohlzylinders abstützt,
e) einem Mitnehmerstift der an der dem Rückstellelement entgegengesetzten Stirnseite des Arbeitskolbens mit dem Arbeitskolben verbunden ist,
f) einem Mitnehmer der mit dem Mitnehmerstift verbunden ist,
g) einem axial im Hohlzylinder verschiebbaren Steuerkolben, der in axialer Achse mindestens so lang ist wie die Summe des axialen Abstands zwischen der Gaseinlassöffnung und der Gasauslassöffnung und der axialen Durchmesser der Gaseinlassöffnung und der Gasauslassöffnung, wobei der Steuerkolben bei axialer Verschiebung über die Gaseinlassöffnung die Gaseinlassöffnung gasdicht verschließt und gleichzeitig die Gasauslassöffnung öffnet, der Steuerkolben bei Verschiebung über die Gasaustrittsöffnung die Gasauslassöffnung gasdicht verschließt und gleichzeitig die Gaseinlassöffnung öffnet, der Steuerkolben mindestens einen Hohlraum besitzt, in dem der Mitnehmerstift axial verschoben werden kann,
h) ein Anschlag am Steuerkolben, auf der dem Arbeitskolben zugewandten Seite des Steuerkolbens so angeordnet ist, dass bei Bewegung des Arbeitskolbens in Richtung des Rückstellelements der Mitnehmerstift durch den Hohlraum des Steuerkolbens bewegt wird und dann der am Mitnehmerstift angeordnete Mitnehmer auf den Anschlag trifft und den Steuerkolben bei weiterer Bewegung des Arbeitskolbens in Richtung Rückstellelement bewegt,
i) einem Anschlag der die axiale Bewegung des Steuerkolbens entgegengesetzt zum Rückstellelement begrenzt und
j) einem gasundurchlässigen Verschluss am dem Rückstellelement entgegengesetztem Ende des Holzylinders, so dass ein Hohlraum durch den Holzylinder gebildet wird, der durch den Arbeitskolben und den gasundurchlässigen Verschluss abgegrenzt wird.

Der Druckgasmotors kann als Antrieb von Pumpen Verwendung finden, bevorzugt als Antrieb von medizinischen Spülsystemen, als Antrieb von Arzneimitteldosierpumpen, als Antrieb von Schmiermittelpumpen, als Antrieb von Dosierpumpen für Pflanzenschutzmittel, als Antrieb von Dosierpumpen für Waschmittel und ganz besonders bevorzugt zum Antrieb von medizinischen Spülsystemen, die nur zum einmaligen Gebrauch bestimmt sind. Daneben kann der Druckgasmotor auch nach Verbindung des Arbeitskolbens mit geeigneten mechanischen Kopplungselementen, wie Pleuelstangen, auch zur Erzeugung von Drehbewegungen verwendet werden. Der Druckgasmotor kann weiterhin zum Antrieb von bewegten Maschinenelementen genutzt werden. Eine Verwendung des Druckgasmotors zum Antrieb von Modellfahrzeugen und Spielzeugen ist ebenfalls möglich.

Ein medizinisches Spülsystem für den einmaligen Gebrauch, bei dem der erfindungsgemäße Druckgasmotor verwendet wird, hat beispielsweise den folgenden Aufbau: Eine Gaspatrone für verflüssigtes Druckgas, eine Öffnungsvorrichtung für die Druckgaspatrone, eine gasdichte Verbindung der Öffnungsvorrichtung mit einem Verdampfungsbehälter für das flüssige Druckgas, eine Gasableitung vom Verdampfungsbehälter, die mit einem Druckreduzierventil verbunden ist, dass über eine weitere Gasleitung mit einem Steuerkolben verbunden ist, das den Gasstrom regulieren kann, das wiederum mit der durch den Druckgasmotor angetriebenen Pumpe verbunden ist, an die wiederum ein Flüssigkeitsaustragsrohr angebracht ist. Die gesamten Elemente des Spülsystems können bis auf das Flüssigkeitsaustragsrohr im Handstück des Systems angeordnet sein und können leicht in einer Hand gehalten werden.

Im Folgenden werden Ausführungsbeispiele der Erfindung anhand von dreizehn schematisch dargestellten Figuren erläutert, ohne jedoch dabei die Erfindung zu beschränken. Dabei zeigt:
- Figur 1:: eine schematische Querschnittansicht eines erfindungsgemäßen Druckgasmotors im Ausgangszustand beziehungsweise der Startposition;
- Figur 2:: eine schematische Querschnittansicht des erfindungsgemäßen Druckgasmotors nach Figur 1, bei dem der Arbeitskolben durch das Druckgas bewegt ist;
- Figur 3:: eine schematische Querschnittansicht des erfindungsgemäßen Druckgasmotors nach Figur 1, bei dem der Mitnehmer des Arbeitskolbens in den Steuerkolben greift und diesen nachzieht;
- Figur 4:: eine schematische Querschnittansicht des erfindungsgemäßen Druckgasmotors nach Figur 1, bei dem der Arbeitskolben und der Steuerkolben maximal in Richtung der Vorderseite ausgelenkt sind;
- Figur 5:: eine schematische Querschnittansicht des erfindungsgemäßen Druckgasmotors nach Figur 1, bei dem der Arbeitskolben durch die Feder wieder entgegengesetzt bewegt ist;
- Figur 6:: eine schematische Querschnittansicht des erfindungsgemäßen Druckgasmotors nach Figur 1, bei dem der Arbeitskolben wieder auf den Steuerkolben trifft und diesen entgegengesetzt bewegt;
- Figur 7:: eine schematische Querschnittansicht eines alternativen erfindungsgemäßen Druckgasmotors mit einer Zugfeder als Rückstellelement und einem Rückschlagventil an der Flüssigkeitszufuhröffnung;
- Figur 8:: eine schematische Querschnittansicht eines weiteren alternativen erfindungsgemäßen Druckgasmotors mit einer Stange zur Bildung eines stoßenden Motors;
- Figur 9:: eine schematische Querschnittansicht eines vierten alternativen erfindungsgemäßen Druckgasmotors mit einer Schnur oder einem Kabel als Mitnehmerelement;
- Figur 10:: eine schematische Querschnittansicht eines fünften alternativen erfindungsgemäßen Druckgasmotors, bei dem das Mitnehmerelement am Steuerkolben befestigt ist;
- Figur 11:: eine schematische Querschnittansicht eines sechsten alternativen erfindungsgemäßen Druckgasmotors, mit einem verkleinerten Arbeitsraum;
- Figur 12:: eine schematische Querschnittansicht eines erfindungsgemäßen Lavage-Systems mit einem erfindungsgemäßen Druckgasmotor; und
- Figur 13:: eine schematische Aufsicht auf ein erfindungsgemäßes Lavage-System mit zwei erfindungsgemäßen Druckgasmotoren.

In den Figuren werden teilweise für gleiche oder gleichartige Bauteile die gleichen Bezugszeichen verwendet.

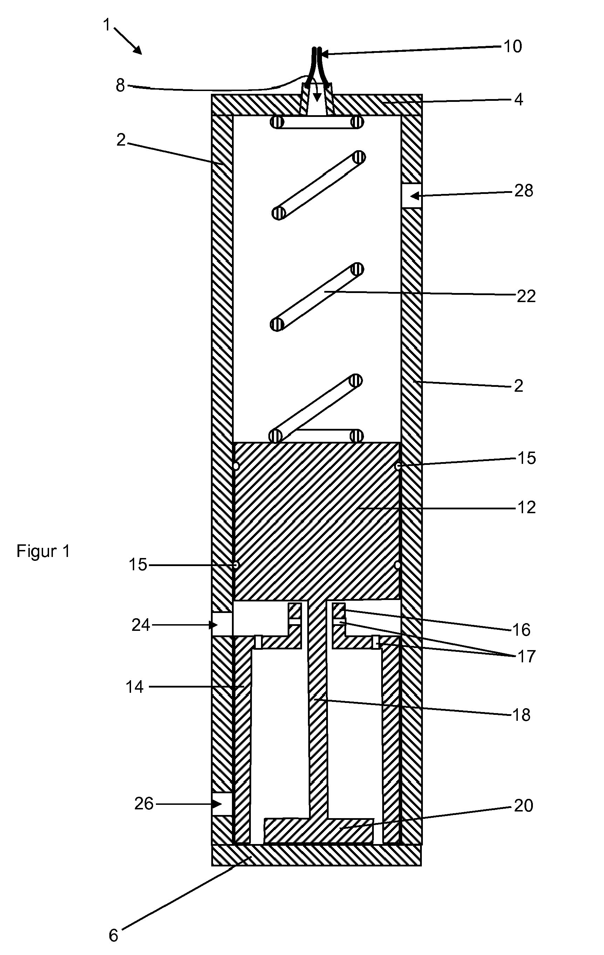

Die Figuren 1 bis 6 zeigen schematische Querschnittansichten eines erfindungsgemäßen Druckgasmotors 1 während eines Arbeitszyklus in chronologischer Abfolge. Die Querschnitte enthalten die Symmetrieachse der Bauteile des Druckgasmotors 1, das heißt, der Querschnitt ist mittig geschnitten dargestellt. Der Druckgasmotor 1 weist einen Hohlkörper 2 aus Kunststoff mit einem zylindrischen Innenraum auf. Der Innenraum ist auf der Vorderseite durch eine Deckplatte 4 und auf der Rückseite durch eine Rückplatte 6 geschlossen. In der Deckplatte 4 ist eine Ausstoßöffnung 8 zum Austreiben eines Flüssigkeitsstrahls vorgesehen.

Die Ausstoßöffnung 8 ist durch eine Lippenventil 10 geschlossen, das sich öffnet, wenn eine Flüssigkeit aus dem Innenraum ausgestoßen wird, das heißt, der Druck im Innenraum ausreichend groß ist. Das heißt, dass der Druck im Innenraum größer ist als der Umgebungsdruck und die elastische Kraft des Lippenventils 10. Die Rückplatte 6 schließt den Innenraum des Hohlkörpers 2 gasdicht und druckdicht ab. In dem Innenraum befinden sich ein entlang der Zylinderachse des zylindrischen Innenraums beweglich angeordneter Arbeitskolben 12 und ein in gleicher Art beweglicher Steuerkolben 14. Der Steuerkolben 14 liegt vollumfänglich dicht an den Zylinderwänden des Innenraums an. Der Arbeitskolben 12 ist durch zwei Dichtringe 15 aus Gummi oder einem anderen elastischen Material gegen die Innenwände des Innenraums abgedichtet. Dazu liegen die Dichtringe 15 vollumfänglich an dem Arbeitskolben 12 und den Innenwänden des Hohlkörpers 2 an.

Der Steuerkolben 14 ist ebenfalls aus Kunststoff gefertigt und hat die Grundform eines Rohrs mit einer Abdeckung. Auf der Abdeckung ist ein rohrförmiger Abstandhalter 16 angeordnet, der in Richtung des Arbeitskolbens 12 weist und über die Wandungen des an den Innenwänden des Innenraums anliegenden Rohrs des Steuerkolbens 14 hinausragt. In der Abdeckung und dem Abstandhalter 16 sind Durchgänge 17 angeordnet, die einen Gasaustausch zwischen den beiden Seiten des Steuerkolbens 14 ermöglichen sollen. Ein weiterer zentraler Durchgang auf der Symmetrieachse ist innerhalb des Abstandhalters 16 vorgesehen.

Der Steuerkolben 14 ist zwischen dem Arbeitskolben 12 und der Rückplatte 6 im Innenraum beweglich angeordnet. An dem Arbeitskolben 12 ist eine Mitnehmereinheit bestehend aus einem Mitnehmerstift 18 und einem Mitnehmer 20 befestigt. Der Mitnehmerstift 18 ist bevorzugt einteilig mit dem Arbeitskolben 12 ausgeführt und ebenfalls aus Kunststoff. Der Mitnehmerstift 18 ist eine zylindrische Stange, die durch den axialen Durchgang im Steuerkolben 14 reicht. Der Mitnehmer 20 ist eine flache Scheibe, die nicht durch den zentralen Durchgang im Abstandhalter 16 des Steuerkolbens 14 passt.

Zwischen der Deckplatte 4 und dem Arbeitskolben 12 ist eine Stahlfeder 22 im Innenraum angeordnet. Die Stahlfeder 22 drückt den Arbeitskolben 12 auf den Abstandhalter 16 am Steuerkolben 14, so dass der Steuerkolben 14 in der gezeigten Position gehalten wird, wenn kein Druckgas (ein Gas mit einem Druck oberhalb des Umgebungsdrucks) durch eine Gaseinlassöffnung 24 in den Innenraum geleitet wird. Die Stahlfeder 22 muss nicht an dem Arbeitskolben 12 und/oder der Deckplatte 4 anliegen. Es reicht auch aus, wenn die Stahlfeder 22 so lange ist, dass sie während des Arbeitsprozesses des Druckgasmotors 1 für eine ausreichende Rückstellkraft auf den Arbeitskolben 12 sorgt. Bevorzugt ist die Stahlfeder 22 jedoch so lange, dass sie in dem in Figur 1 dargestellten Ausgangszustand den Arbeitskolben 12 auf den Steuerkolben 14 und diesen an die Rückplatte 6 drückt. Dadurch kann nämlich sichergestellt werden, dass die Gaseinlassöffnung 24 offen bleibt, wenn kein Druckgas in den Druckgasmotor 1 eingespeist wird, und der Druckgasmotor 1 immer in den Ausgangszustand überführt wird.

Die Gaseinlassöffnung 24 ist eine durchgehende Öffnung in der Seitenwand des Hohlkörpers 2. Neben der Gaseinlassöffnung 24 sind in der Seitenwand des Hohlkörpers 2 noch eine Gasauslassöffnung 26 und eine Flüssigkeitszufuhröffnung 28 vorgesehen. Die Gasauslassöffnung 26 ist in dem Ausgangszustand des Druckgasmotors 1, wie er in Figur 1 gezeigt ist, durch den Steuerkolben 14 verschlossen beziehungsweise abgedeckt. Durch die Gasauslassöffnung 26 wird das Arbeitsmedium (das Druckgas) aus dem Innenraum abgeblasen, wenn die Gasauslassöffnung 26 durch eine Bewegung des Steuerkolbens 14 freigelegt wird. Die Flüssigkeitszufuhröffnung 28 ist dagegen zwischen dem Förderkolben 12 und der Deckplatte 4 angeordnet und ist dazu vorgesehen, den Innenraum in diesem Bereich mit einer Flüssigkeit zu füllen, die dann bei einer Bewegung des Arbeitskolbens 12 auf die Deckplatte 4 zu durch die Ausstoßöffnung 8 ausgepresst wird und so einen Sprühstrahl (nicht gezeigt) erzeugt.

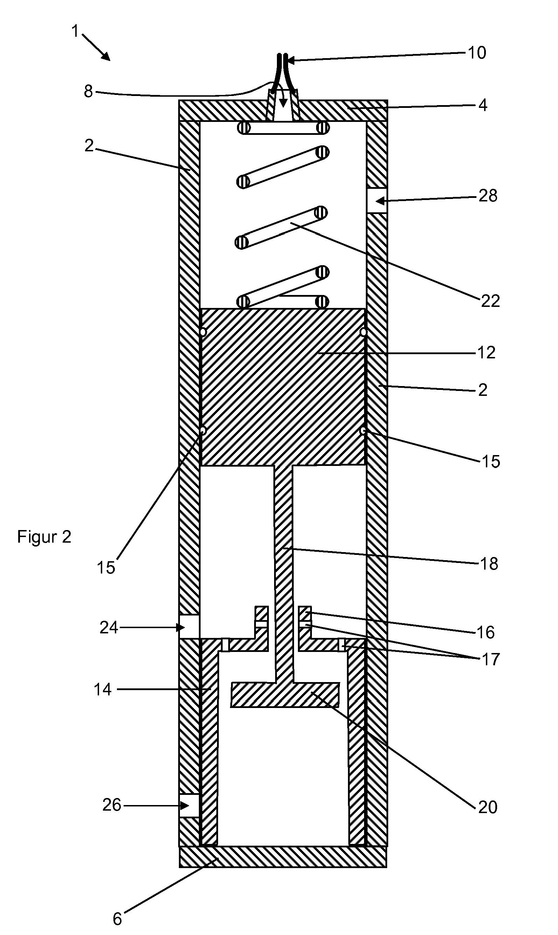

In Figur 1 ist der Ausgangszustand des Druckgasmotors 1 gezeigt. Ein Druckgas wird über die Gaseinlassöffnung 24 in den Innenraum zwischen den Arbeitskolben 12 und den Steuerkolben 14 eingespeist. Der in diesem Zwischenraum zunehmende Druck beschleunigt den Arbeitskolben 12 in Richtung der Deckplatte 4, also der Vorderseite des Druckgasmotors 1. Der Steuerkolben 14 bleibt dabei an Ort und Stelle. Diese Situation ist in Figur 2 dargestellt. Sofern eine Flüssigkeit in dem Innenraum zwischen dem Arbeitskolben 12 und der Deckplatte 4 vorhanden ist, wird diese durch die Bewegung des Arbeitskolbens 12 durch die Ausstoßöffnung 8 ausgestoßen. Der Flüssigkeitsausstoß wird so lange stattfinden, wie sich der Arbeitskolben 12 in Richtung der Deckplatte 4 bewegt.

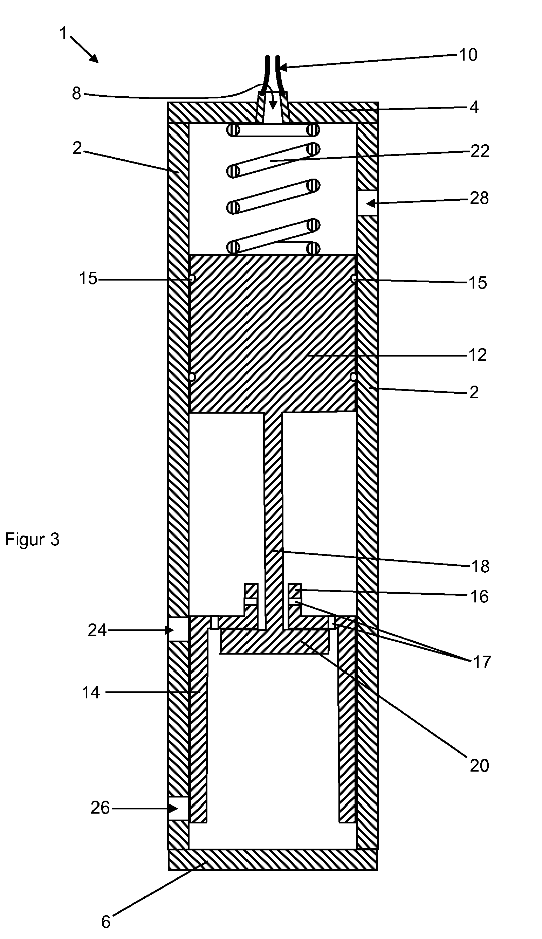

Während der Bewegung des Arbeitskolbens 12 drückt dieser das Federelement 22 zusammen. Wenn sich der Arbeitskolben 12 so weit von dem Steuerkolben 14 entfernt hat, dass die Entfernung der Länge des Mitnehmerstifts 18 abzüglich der Dicke der Abdeckung des Steuerkolbens 14 entspricht, trifft der Mitnehmer 20 auf die Rückseite der Abdeckung des Steuerkolbens 14 und schlägt diesen nach vorne. Der Steuerkolben 14 wird dann von dem Arbeitskolben mit Hilfe des Mitnehmerelements 18, 20 mitgezogen. Dadurch bewegt sich der Steuerkolben 14 von der Rückplatte 6 weg. Durch die Bewegung des Steuerkolbens 14 wird die Gaseinlassöffnung 24 abgedeckt beziehungsweise geschlossen. Diese Situation ist in Figur 3 dargestellt.

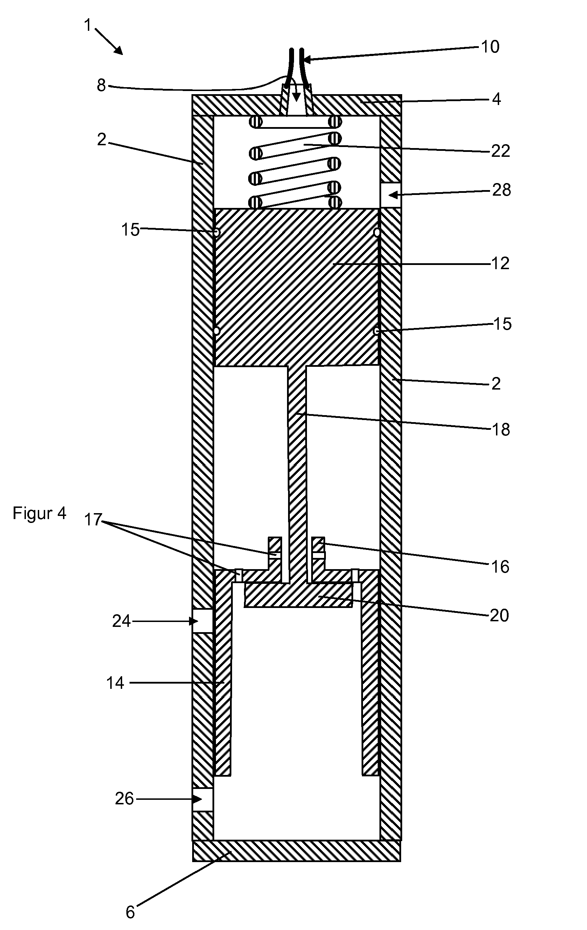

Während der ganzen Zeit wird der Inhalt des Innenraums zwischen dem Arbeitskolben 12 und der Deckplatte 4 durch die Ausstoßöffnung ausgestoßen. Durch die Trägheit des Arbeitskolbens 12 beziehungsweise des Steuerkolbens 14 und/oder dem immer noch zwischen dem Arbeitskolben 12 und der Rückplatte 6 existierenden Überdrucks im Innenraum wird der Steuerkolben 14 noch weiter nach vorne bewegt und die Gasauslassöffnung 26 geöffnet. Diese Situation ist in Figur 4 dargestellt.

Anschließend strömt das Gas aus dem Innenraum durch die Gasauslassöffnung 26 heraus und wird an die Umgebung abgegeben. Das Federelement 22 beschleunigt den Arbeitskolben 12 in Richtung der Rückplatte 6. Durch das sich vergrößernde Volumen zwischen dem Arbeitskolben 12 und der Deckplatte 4 entsteht dort ein Unterdruck und das Lippenventil 10 schließt. Durch die Flüssigkeitszufuhröffnung 28 strömt neue Flüssigkeit in den sich vergrößernden Zwischenraum zwischen dem Arbeitskolben 12 und der Deckplatte 4. Bevorzugt ist an der Flüssigkeitszufuhröffnung 28 ein Rückschlagventil (nicht gezeigt) angeordnet, das ein Austreten der Flüssigkeit aus dem Zwischenraum zwischen dem Arbeitskolben 12 und der Deckplatte 4 in die Flüssigkeitszufuhr verhindert.

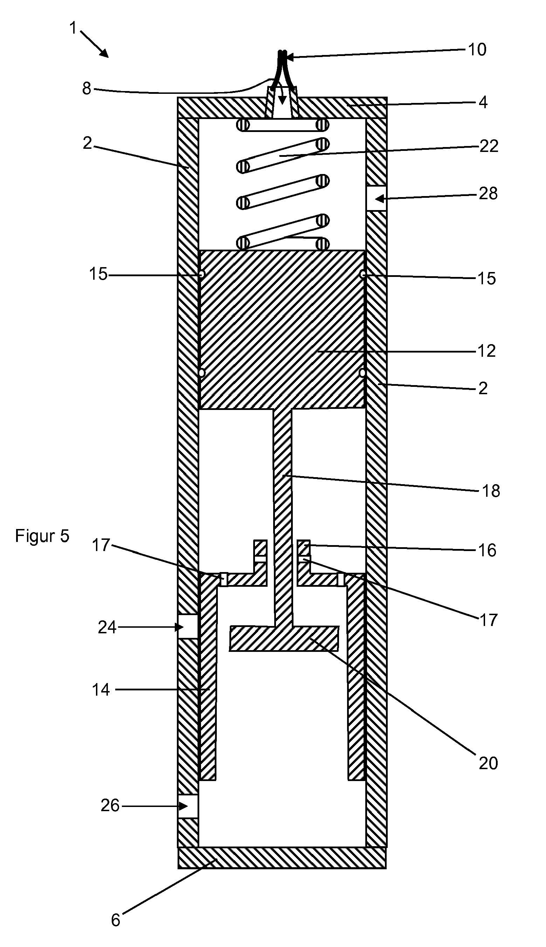

Der Arbeitskolben 12 löst durch seine Bewegung den Mitnehmer 20 von der Rückseite der Abdeckung des Steuerkolbens 14. Diese Situation ist in Figur 5 gezeigt.

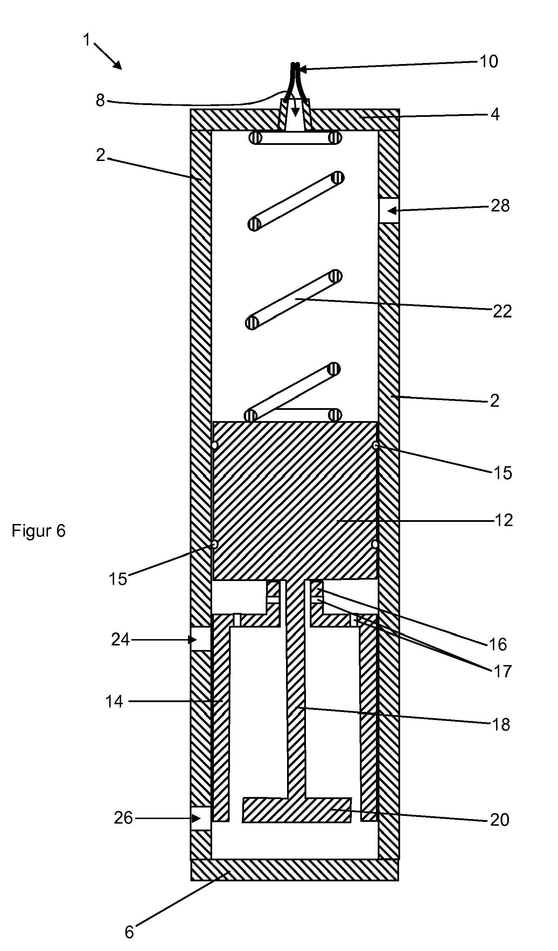

Der Arbeitskolben 12 trifft schließlich auf den Steuerkolben 12 beziehungsweise auf den Abstandhalter 16 des Steuerkolbens 12. Dadurch wird auch der Steuerkolben 12 wieder in Richtung der Rückplatte 6 bewegt und die Gasauslassöffnung 26 beginnt sich zu schließen. Dieser Zustand ist in Figur 6 gezeigt.

Schließlich öffnet sich auch wieder die Gaseinlassöffnung 24 und das Federelement 22 hat den Arbeitskolben 12 und den Steuerkolben 14 wieder in den in Figur 1 gezeigten Ausgangszustand überführt. Währenddessen hat sich der Zwischenraum zwischen dem Arbeitskolben 12 und der Deckplatte 4 wieder mit Flüssigkeit oder einem Flüssigkeit-Gas-Gemisch aus der Flüssigkeitszufuhröffnung 28 gefüllt. Der Zyklus beginnt von vorn.

Der Steuerkolben 14 und dessen Bewegung bewirken eine automatische Ventilsteuerung der Gaseinlassöffnung 24 und der Gasauslassöffnung 26, so dass der Steuerkolben 14 als Ventilelement aufgefasst werden kann.

Durch Einwirkung des Druckgases wird bei der ersten Bewegung des Arbeitskolbens 12 in Richtung des Rückstellelements 22 die im Hohlraum vorhandene Luft durch die Ausstoßöffnung 8 und das Auslassventil 10 heraus gepresst. Ein Rückschlagventil (nicht gezeigt), das an der Flüssigkeitszufuhröffnung 28 angeordnet ist, verhindert ein Austritt der Luft in das Flüssigkeitsreservoir. Nach Öffnung der Gasauslassöffnung 26 und der Bewegung des Arbeitskolbens 12 in Richtung der Ausgangslage wird durch die Kraft des Rückstellelements 22 ein Unterdruck im Hohlraum erzeugt. Dabei öffnet sich das Rückschlagventil und die Flüssigkeit fließt in den Hohlraum. Nach Schließen der Gasauslassöffnung 26 bewegt sich durch Einwirkung des Druckgases der Arbeitskolben 12 in Richtung Rückstellelement 22. Dabei wird die Flüssigkeit durch das Auslassventil 10 heraus gepresst, wobei gleichzeitig das Rückschlagventil geschlossen ist. Nach Öffnung der Gasauslassöffnung 8 drückt das Rückstellelement 22 den Arbeitskolben 12 zurück in seine Ausgangslage, wobei durch den Unterdruck das Rückschlagventil öffnet und Flüssigkeit in den Innenraum gesaugt beziehungsweise gedrückt wird. Danach läuft der Pumpprozess der Flüssigkeit in der beschriebenen Weise solange ab, wie Druckgas an der Gaseinlassöffnung 24 anliegt und sich Flüssigkeit im Flüssigkeitsreservoir befindet.

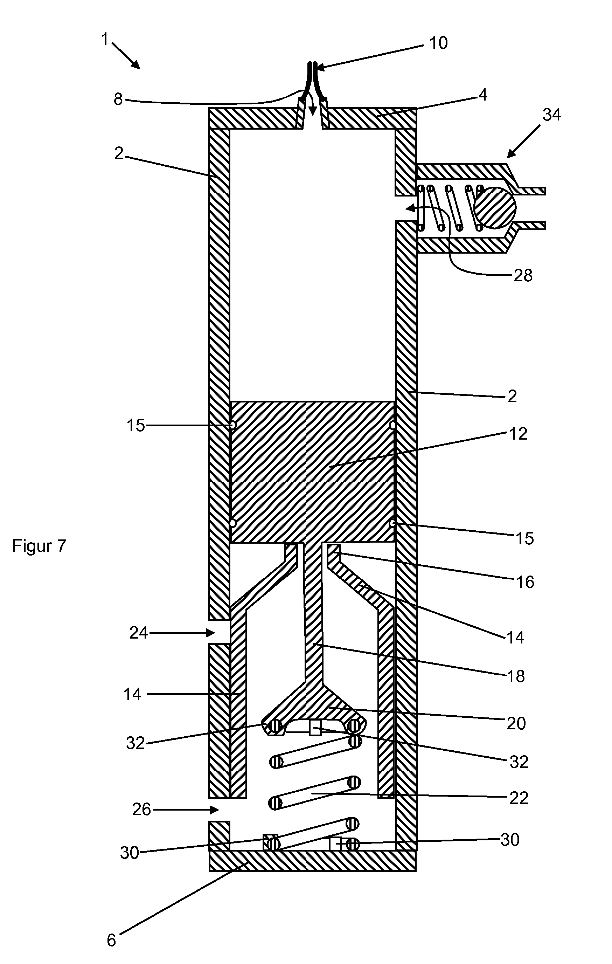

Figur 7 zeigt eine schematische Querschnittansicht eines alternativen erfindungsgemäßen Druckgasmotors 1 mit einer Zugfeder 22 als Rückstellelement 22, die über Halterungen 30 mit einer Rückplatte 6 verbunden ist und auf der gegenüberliegenden Seite über Halterungen 32 mit einem Mitnehmer 20 verbunden ist. Zudem weist der Druckgasmotor 1 ein Rückschlagventil 34 auf. Zudem ist die Form des Mitnehmers 20 sowie der Eingriff in den Steuerkolben 14 vorliegend konisch gestaltet. Die Durchführung durch den Steuerkolben 14, durch die sich der Mitnehmerstift 18 erstreckt, dient vorliegend auch dem Gasaustausch des Zwischenraums zwischen dem Arbeitskolben 12 und dem Steuerkolben 14 und dem Zwischenraum zwischen dem Steuerkolben 14 und der Rückplatte 6. Auf zusätzliche Durchführungen wird bei dieser Ausführungsform verzichtet. Ansonsten gleichen der Aufbau und die Funktionsweise denen, die in den Figuren 1 bis 6 dargestellt und vorstehend beschrieben sind.

Die Zugfeder 22 zieht den Arbeitskolben 12 so weit in Richtung der Rückplatte 6, dass der Steuerkolben 14 an der Rückplatte 6 anliegt und der Arbeitskolben 12 am Steuerkolben 14 anliegt. Bevorzugt übt die Zugfeder 22 auch dann noch eine Kraft auf den Arbeitskolben 12 aus, die den Arbeitskolben 12 in Richtung der Rückplatte 6 zieht. Die Arbeitsweise des Druckgasmotors 1 nach Figur 7 entspricht der Funktionsweise des Druckgasmotors 1 nach den Figuren 1 bis 6, nur dass die Feder 22 nach Figur 7 durch Dehnung gespannt wird, also eine Zugfeder ist, während die Feder 22 nach den Figuren 1 bis 6 durch Stauchen potentielle Energie aufnimmt.

Das Rückschlagventil 34, das in der gleichen Form auch an den Flüssigkeitszufuhröffnungen 28 anderer Druckgasmotoren, wie dem nach den Figuren 1 bis 6 angeordnet sein kann, besteht aus einer Kugel, die mit einer Stahlfeder auf einen Kugelsitz gepresst wird. Dadurch wird eine Strömung in den Innenraum des Hohlkörpers 2 hinein durch die Flüssigkeitszufuhröffnung 28 ermöglicht, während eine Strömung aus dem Innenraum heraus durch das Rückschlagventil 34 blockiert wird. Wenn also der Inhalt des Druckgasmotors 1 zwischen dem Arbeitskolben 12 und der Deckplatte 4 durch eine Bewegung des Arbeitskolbens 12 auf die Deckplatte 4 zu durch die Ausstoßöffnung 8 ausgesprüht wird, kann der Inhalt wegen des Rückschlagventils 34 nicht in die Zuleitung vordringen.

Die druckgasgetriebene Flüssigkeitspumpe 1 funktioniert in der Weise, dass auf der Stirnseite des Arbeitskolbens 12, an dem das Rückstellelement 22 angreift, ein Hohlraum vorhanden ist. Dieser Hohlraum ist mit dem Rückschlagventil 34 flüssigkeitsdurchlässig verbunden, das einen Flüssigkeitseinstrom aus einem Flüssigkeitsreservoir in den Hohlraum ermöglicht und ein Zurückströmen der Flüssigkeit aus dem Hohlraum in Richtung Flüssigkeitsreservoir verhindert. Weiterhin ist ein Auslassventil 10 flüssigkeitsdurchlässig mit dem Hohlraum verbunden. Dieses ermöglicht ein Ausströmen der Flüssigkeit aus dem Hohlraum und verhindert ein Zurückströmen der Flüssigkeit zurück in den Hohlraum. Dieses Auslassventil 10 kann im einfachsten Fall ein Lippenventil 10 sein. Das Rückstellelement 22 kann sich alternativ auch im Hohlraum befinden (siehe Figur 1 bis 6). Die grundsätzliche Funktionsweise des Druckgasmotors 1 erfolgt in der beschriebenen Weise.

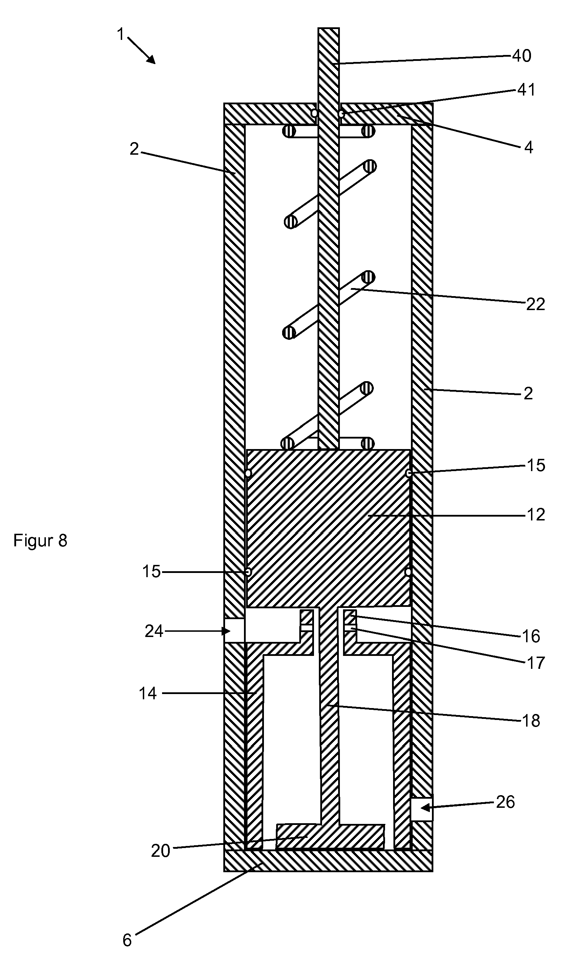

Figur 8 zeigt eine schematische Querschnittansicht eines dritten alternativen erfindungsgemäßen Druckgasmotors 1 mit einer Stange 40 zur Bildung eines stoßenden Druckgasmotors 1. Der Aufbau des Druckgasmotors 1 gleicht dem der zuvor beschriebenen Druckgasmotoren nach den Figuren 1 bis 6 und 7. Der Druckgasmotor 1 weist einen Hohlkörper 2 mit einem zylindrischen Innenraum auf. Der Innenraum ist auf der Vorderseite mit einer Deckplatte 4 abgedeckt und auf der Rückseite mit einer Rückplatte 6 gas- und druckdicht abgeschlossen. Im Innenraum ist ein Arbeitskolben 12 linear beweglich entlang der Zylinderachse des Innenraums angeordnet. Zwischen dem Arbeitskolben 12 und der Rückplatte 6 ist ein Steuerkolben 14 linear beweglich entlang der Zylinderachse des Innenraums angeordnet. Arbeitskolben 12 und Steuerkolben 14 weisen einen zylindrischen Außenmantel auf, der dem inneren Zylindermantel des Innenraums entspricht.

Der Arbeitskolben 12 ist mit zwei Dichtungsringen 15 gegen die Innenwand des Innenraums abgedichtet. An der Vorderseite des Steuerkolbens 14 ist eine zylindrische Hülse als Abstandhalter 16 angeordnet. In den Wänden des Abstandhalters sind Durchgänge 17 angeordnet, die einen Gasaustausch zwischen der Vorderseite und der Rückseite des Steuerkolbens 14 ermöglichen.

An dem Arbeitskolben 12 ist auf der Rückseite ein Mitnehmerelement angeordnet, das aus einem Mitnehmerstift 18 und einem Mitnehmer 20 besteht. Der Mitnehmerstift 18 reicht durch eine Durchführung im Steuerkolben 14, so dass der Mitnehmer 20 auf der Rückseite des Steuerkolbens 14 eingreifen kann, um den Steuerkolben 14 beabstandet zum Arbeitskolben 12 mitzuziehen, wenn sich der Arbeitskolben 12 in Richtung der Vorderseite, also der Deckplatte 4 bewegt.

In dem Innenraum zwischen dem Arbeitskolben 12 und der Deckplatte 4 ist ein Federelement 22 um die Stange 40 herum angeordnet, die den Arbeitskolben 12 in Richtung der Rückplatte 6 und in Figur 8 auf den Steuerkolben 14 drückt. In Figur 8 ist also die Ausgangsstellung des Druckgasmotors 1 gezeigt.

In der Seitenwand des Hohlkörpers 2 sind zwei durchgehende Öffnungen 24, 26 vorgesehen. An der Gaseinlassöffnung 24 wird ein Druckgas in den Druckgasmotor 1 eingespeist. Während des Arbeitszyklus des Druckgasmotors 1 wird das Druckgas durch die Gasauslassöffnung 26 wieder ausgestoßen. Der Arbeitszyklus läuft ab, wie mit dem ersten Ausführungsbeispiel nach den Figuren 1 bis 6 beschrieben.

Anstatt eine Flüssigkeit periodisch auszustoßen, wie mit dem Lavage-System nach den Figuren 1 bis 6, wird mit dem Druckgasmotor 1 ein periodisches Stoßen der Stange 40 erreicht. Die Stange 40 ist mit einer Führungshülse 41 durch eine Öffnung in der Deckplatte 4 aus dem Innenraum heraus durchgeführt. Alternativ kann anstatt der starr mit dem Arbeitskolben 12 verbundenen Stange 40 kann auch eine Pleuelstange 40 über ein Gelenk (nicht gezeigt) mit dem Arbeitskolben 12 verbunden sein, der durch die Deckplatte 4 durchgeführt ist. Mit der Pleuelstange 40 kann die lineare periodische Bewegung mit Hilfe einer Kurbelwelle (nicht gezeigt), die vorne an der Pleuelstange 40 über ein Gelenk befestigt ist, in eine Drehbewegung übersetzt werden.

Der Druckgasmotor 1 nach Figur 8 kann ebenfalls in einem Lavage-System eingesetzt werden. Dazu wird die Spitze der Stange 40 dazu eingesetzt, auf eine Membran (nicht gezeigt) zu stoßen. Die periodischen Druckstöße auf die Membran können zum periodischen Ausstoß einer Flüssigkeit verwendet werden. Ebenso kann die periodische Bewegung der Stange 40 als Antrieb für einen Rüttelmechanismus oder einen Klopfermotor verwendet werden.

Alternativ kann der Druckgasmotor 1, wenn er mit einer Pleuelstange 40 ausgeführt ist, zum Antrieb einer Trennscheibe oder eines Spielzeugzeugs oder einer Pumpe verwendet werden.

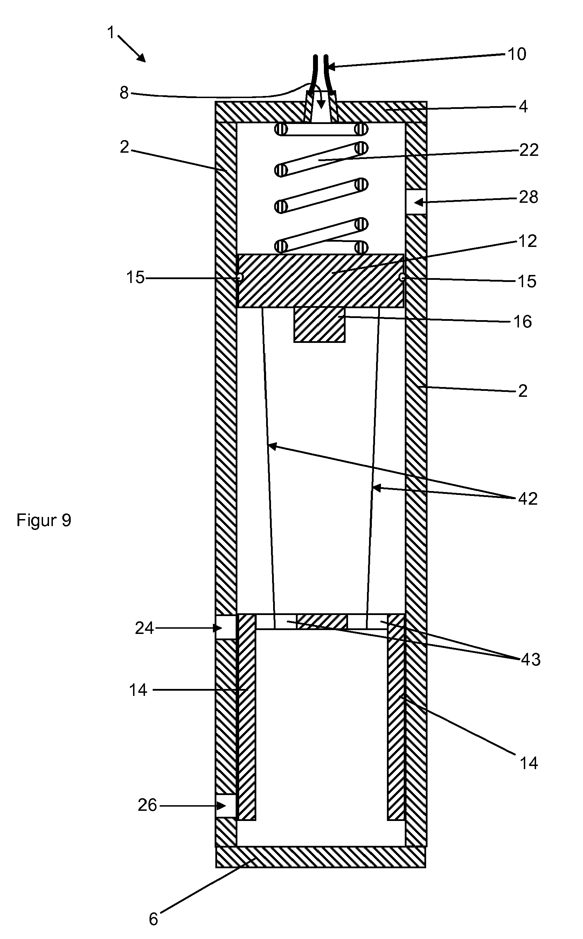

Figur 9 zeigt eine schematische Querschnittansicht eines vierten alternativen erfindungsgemäßen Druckgasmotors 1 mit einer Schnur 42 oder einem Kabel 42 als Mitnehmerelement 42. Bei dieser Version des Druckgasmotors 1 ist der Abstandhalter 16 auf der Rückseite des Arbeitskolbens 12 angeordnet. Der Arbeitskolben 12 ist vorliegend nur über einen O-Ring 15 gegen die Innenwände des zylindrischen Innenraums im Hohlkörper 2 abgedichtet.

Bei der in Figur 9 gezeigten Stellung des Druckgasmotors 1 sind die Kabel 42 gerade gespannt. Der Arbeitskolben 12 zieht den Steuerkolben 14 hinter sich her in Richtung der Deckplatte 4, um die Gaseinlassöffnung 24 zu schließen und die Gasauslassöffnung 26 danach zu öffnen. Die Kabel 42 sind an Streben 43 an der Vorderseite des Steuerkolbens 14 befestigt, beispielsweise mit einer Schlaufe um die Streben 43 herum gebunden.

Wenn der Arbeitskolben 12 mit dem Federelement 22 in Richtung der Rückplatte 6 gedrückt wird, hängen die Kabel 42 lose im Innenraum zwischen dem Arbeitskolben 12 und dem Steuerkolben 14 herum. Nur beim Nachziehen des Steuerkolbens 14 durch den Arbeitskolben 12 sind die Kabel 42 oder Schnüre 42, wie in Figur 9 gezeigt, straff gespannt.

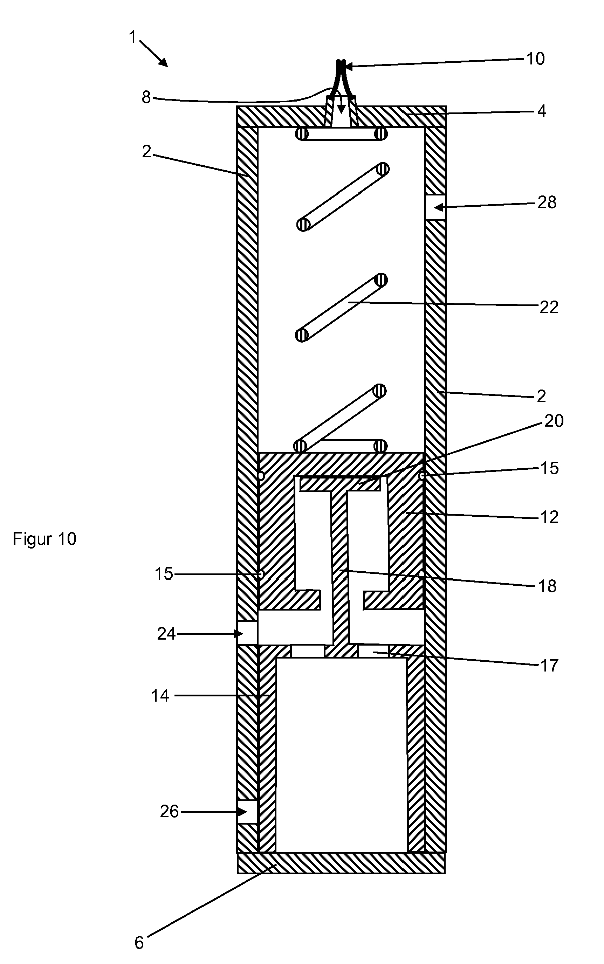

Figur 10 zeigt eine schematische Querschnittansicht eines fünften alternativen erfindungsgemäßen Druckgasmotors 1, bei dem das Mitnehmerelement 18, 20 am Steuerkolben 14 befestigt ist. Das Mitnehmerelement 18, 20 läuft bei dieser Ausführungsform in einem Hohlraum im Arbeitskolben 12. Da der Mitnehmerstift 18 länger ist als die Tiefe des Hohlraums im Arbeitskolben 12, übernimmt er auch die Aufgabe eines Abstandhalters mit dem sichergestellt wird, dass der Arbeitskolben 12 vom Steuerkolben 14 in jedem Fall beabstandet ist, so dass in der in Figur 10 gezeigten Ausgangsstellung das Druckgas durch die Gaseinlassöffnung 24 zwischen den Arbeitskolben 12 und den Steuerkolben 14 strömen kann, um den Arbeitskolben 12 in Richtung der Deckplatte 4 zu beschleunigen.

Die Durchgänge 17 im Steuerkolben 14 sind notwendig, damit das Druckgas im Druckgasmotor 1 auf die Rückseite des Steuerkolbens 14 strömen kann, um schließlich durch die Gasauslassöffnung 26 auszuströmen, wenn diese durch den Steuerkolben 14 geöffnet ist.

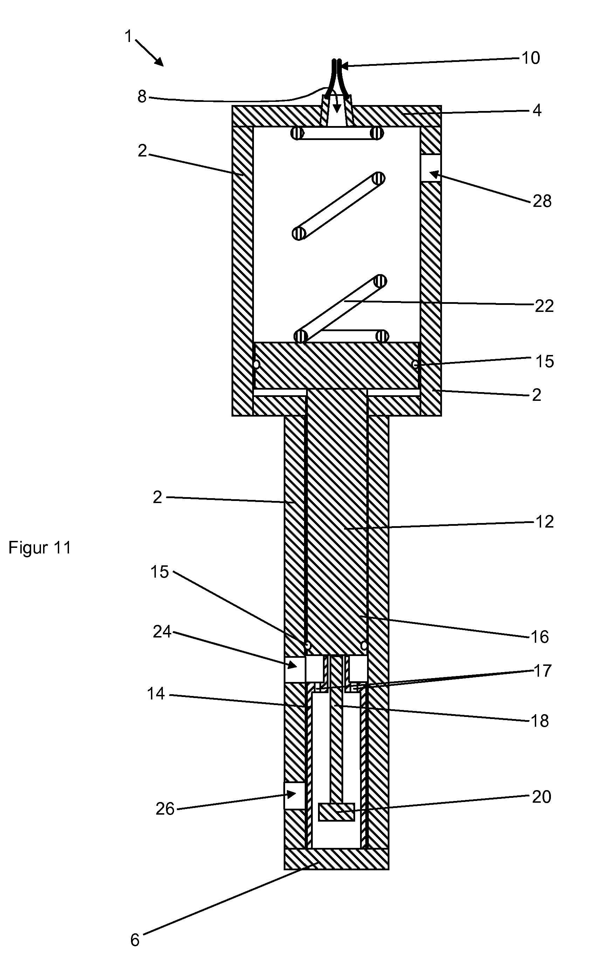

Figur 11 zeigt eine schematische Querschnittansicht eines sechsten alternativen erfindungsgemäßen Druckgasmotors 1, mit einem im Vergleich zu den bisherigen Ausführungsformen verkleinerten Arbeitsraum zwischen einem Arbeitskolben 12 und einem Steuerkolben 14. Der Arbeitsraum ist durch die Verringerung des Innendurchmessers des Hohlkörpers 2 und der Kolben 12, 14 reduziert. Dazu muss der Arbeitskolben 12 zwei verschiedene Querschnitte (oder bei zylindrischem Aufbau mit kreisrunder Grundfläche zwei verschiedene Durchmesser) aufweisen, die zu den Querschnitten der beiden zylindrischen Innenräume passen. Der verkleinerte Arbeitsraum führt dazu, dass weniger Druckgas pro Bewegungszyklus des Druckgasmotors 1 verbraucht wird. Dafür ist die Bewegung etwas weniger kräftig. Dies reicht aber für die Verwendung als Sprüh-Pumpe für ein Lavage-System aber aus.

Der erfindungsgemäße zylindrische Innenraum, in dem sich der Arbeitskolben 12 bewegt, ist hier der mit dem geringen Querschnitt, da dort die Bewegung des Arbeitskolbens 12 durch das Druckgas entsteht. Der Arbeitszyklus und der Pumpvorgang beziehungsweise Sprühvorgang unterscheiden sich nicht von dem zu den Figuren 1 bis 6 beschriebenen Arbeitszyklus. Figur 11 zeigt wieder den Ausgangszustand des Druckgasmotors 1 beziehungsweise des Arbeitskolbens 12 und des Steuerkolbens 14 darin. An der Flüssigkeitszuführöffnung 28 ist wieder ein Rückschlagventil (nicht gezeigt) angeordnet, mit dem ein Eindringen von Flüssigkeit aus dem Pumpraum (vorderer Teil des durch den Hohlkörper gebildeten Raums) in die Flüssigkeitszufuhr verhindert wird.

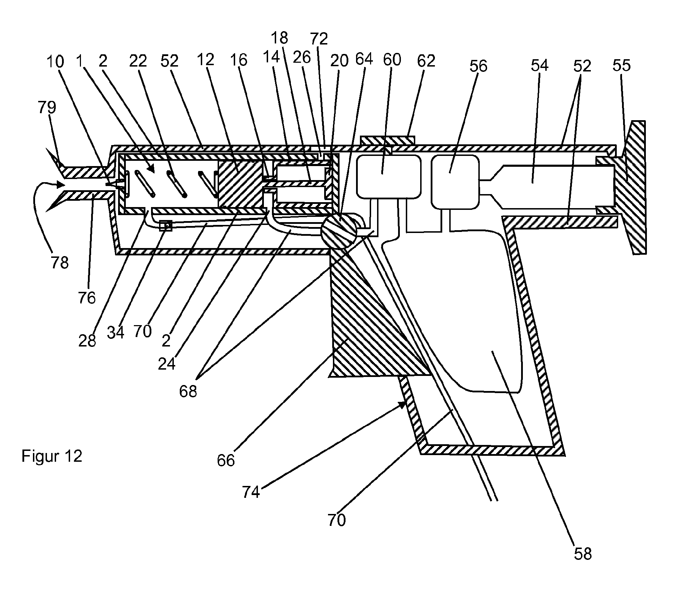

Figur 12 zeigt eine schematische Querschnittansicht eines erfindungsgemäßen in einer Hand haltbaren Lavage-Systems 50 mit einem erfindungsgemäßen Druckgasmotor 1. Der Druckgasmotor 1 ist bis auf die Anordnung des Gaseinlassöffnung 24, der Gasauslassöffnung 26 und der Flüssigkeitszufuhröffnung 28 relativ zueinander wie der Druckgasmotor 1 nach den Figuren 1 bis 6 aufgebaut, so dass für diesen Aufbau auf dieses Ausführungsbeispiel verwiesen werden kann.

Das Lavage-System 50 weist ein Gehäuse 52 aus Kunststoff auf, in dem der Druckgasmotor 1 angeordnet ist. In dem Gehäuse 52 ist in einer Öffnung auf der Rückseite (in Figur 12 rechts) eine Druckgaspatrone 54 angeordnet, in der ein komprimiertes Druckgas oder ein verflüssigtes Druckgas wie zum Beispiel CO₂ enthalten ist, mit dem der Druckgasmotor 1 angetrieben wird. Rückseitig ist an der Druckgaspatrone 54 ein Griff 55 angeordnet, mit dem die Druckgaspatrone 54 in das Lavage-System hineinschraubbar oder hineindrückbar ist. Die Gaspatrone 54 ist eine CO₂-Patrone aus Metall und wird mit dem Griff 55 auf einen Dorn (nicht gezeigt) einer Verbindungseinheit 56 gedrückt, so dass der Dorn die Druckgaspatrone 54 öffnet und das Druckgas aus der Druckgaspatrone 54 in die Verbindungseinheit 56 strömt.

Das verflüssigte Gas aus der Druckgaspatrone 54 strömt anschließend durch eine Druckgasleitung von der Verbindungseinheit 56 in einen Verdampfungsbehälter 58, in dem flüssige Bestandteile des Gases verdampfen können. Der Verdampfungsbehälter 58 ist wiederum über eine Druckgasleitung mit einem Druckminderungsventil 60 verbunden. Das Druckminderungsventil 60 kann über eine Einstellschraube 62 an der Oberseite des Lavage-Systems bedient werden. Mit dem Druckminderungsventil 60 kann der zum Betreiben des Druckgasmotors 1 gewünschte Gasdruck eingestellt werden.

Das Druckgas wird von dem Druckminderungsventil 60 über eine Gaszuleitung 68 zu einem Steuerungsventil 64 weitergeleitet. Das Steuerungsventil 64 kann mit einem Abzug 66 nach Art einer Pistole manuell mit der gleichen Hand bedient werden, mit dem das Lavage-System gehalten wird. Die Gaszuleitung 68 setzt sich hinter dem manuell bedienbaren Steuerungsventil 64 fort und ist an die Gaseinlassöffnung 24 des Druckgasmotors 1 angeschlossen.

Die Flüssigkeitszufuhröffnung 28 ist an eine Spülflüssigkeitszuleitung 70 angeschlossen in der ein Rückschlagventil 34 angeordnet ist. Auf der Oberseite des Gehäuses 52 ist eine Öffnung 72 vorgesehen, durch die das Druckgas aus dem Druckgasmotor 1, das durch die Gasauslassöffnung 26 entweicht, austreten kann. Die Öffnung 72 kann mit einem Gitter und/oder einem Filter abgedeckt sein.

Der Verdampfungsbehälter 58 ist in einem Griffstück 74 nach Art eines Pistolengriffs angeordnet, der durch das Kunststoffgehäuse 52 gebildet wird. An der Vorderseite des Lavage-Systems ist ein Rohr 76 mit einer Austragsöffnung 78 und einem Trichter 79 angeordnet, durch die die Flüssigkeitsstöße aus dem Druckgasmotor 1 aus dem Lavage-System austreten.

Bevorzugt ist bei solchen Lavage-Systemen eine Absaugeinrichtung (nicht gezeigt) vorgesehen, mit der überschüssige Flüssigkeit und mit der Flüssigkeit abgetragene Teile abgesaugt und abgeleitet werden. Sobald eine Druckgaspatrone 54 in das Lavage-System eingesetzt wird, ist das Lavage-System betriebsbereit. Wenn Steuerungsventil 64 mit dem Abzug bedient wird, wird die Gaszuleitung 68 durchgehend und das Druckgas aus dem Druckminderer 60 wird in den Druckgasmotor 1 eingespeist. Durch das Druckgas werden der Arbeitskolben 12 und anschließend der Steuerkolben 14 in Bewegung gesetzt und der Druckgasmotor 1 arbeitet, wie zuvor beschrieben. Gleichzeitig strömt die Flüssigkeit, die durch die Flüssigkeitszuleitung 70 aus einem externen Flüssigkeitsresevoir (nicht gezeigt) gefördert wird, periodisch in den Zwischenraum zwischen dem Arbeitskolben 12 und der Deckplatte 6 des Druckgasmotors 1, indem sich auch die Feder 22 als Rückstellelement 22 befindet.

Die Flüssigkeit wird aus dem Druckgasmotor 1 mit periodischen Stößen durch das Rohr 76 und die Austragsöffnung 78 ausgestoßen, solange das Druckgas durch die Gaseinlassöffnung 24 in den Druckgasmotor 1 und durch diesen hindurch strömt. Der Prozess wird beendet, wenn die Druckgaspatrone 54 leer ist oder das Steuerungsventil 64 nicht mehr bedient wird und dadurch die Druckgaszuleitung 68 unterbrochen wird. Die Rückstellung des Steuerungsventils 64 erfolgt beispielsweise mit Hilfe einer elastischen Feder. Durch das Federelement 22 im Druckgasmotor 1 werden der Arbeitskolben 12 und der Steuerkolben 14 dann in die gezeigte Ausgangsstellung (siehe auch Figur 1) gebracht und das Lavage-System ist sofort wieder einsatzbereit.

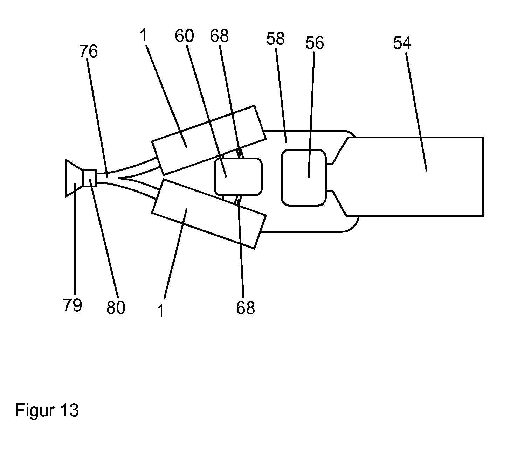

Anstatt nur einen Druckgasmotor 1 kann ein Lavage-System 50 auch zwei oder mehr Druckgasmotoren 1 aufweisen, deren Druckgasanschlüsse parallel geschaltet sind. Hierdurch wird eine Verstärkung des erzeugten Sprühstrahls bewirkt oder ein höher gepulster Ausstoß mit doppelter Frequenz erreicht. Ein solches Lavage-System ist in einer schematischen Aufsicht in Figur 13 gezeigt. Das Lavage-System nach Figur 13 weist zwei Druckgasmotoren 1 auf, die bezogen auf zwei Druckgaszuleitungen 68 parallel geschaltet sind. Die Druckgaszuleitungen 68 werden über einen Druckminderer 60 mit Druckgas versorgt. Dazu ist der Druckminderer 60 mit einer Druckgaspatrone 54 über eine Verbindungseinheit 56 und einen Verdampfungsbehälter 58 verbunden.

Die beiden Druckgasmotoren 1 münden über jeweils ein Rohr 76 in einen Trichter 79, aus dem die Flüssigkeitsströme ausgetragen werden. An dem Trichter 79 ist eine Absaugeinrichtung 80 vorgesehen, mit der Flüssigkeit und mit der Flüssigkeit ausgespülte Reste abgesaugt werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Druckgasmotor
- 2: Hohlkörper
- 4: Deckplatte
- 6: Rückplatte
- 8: Ausstoßöffnung
- 10: Lippenventil
- 12: Arbeitskolben
- 14: Steuerkolben
- 15: Dichtung / O-Ring
- 16: Abstandhalter
- 17: Durchgang
- 18: Mitnehmerstift
- 20: Mitnehmer
- 22: Federelement
- 24: Gaseinlassöffnung
- 26: Gasauslassöffnung
- 28: Flüssigkeitszufuhröffnung
- 30, 32: Halterung
- 34: Rückschlagventil
- 40: Stange / Pleuelstange
- 41: Führungshülse
- 42: Kabel / Schnur
- 43: Strebe
- 50: Lavage-System
- 52: Gehäuse
- 54: Gasdruckpatrone
- 55: Griff für Gasdruckpatrone
- 56: Verbindungseinheit für Gaspatrone
- 58: Verdampfungsbehälter
- 60: Druckreduzierventil
- 62: Einstellschraube
- 64: Steuerungsventil
- 66: Abzug
- 68: Gaszuleitung
- 70: Spülflüssigkeitszuleitung
- 72: Öffnung
- 74: Griffstück
- 76: Rohr
- 78: Austragöffnung
- 80: Absaugeinrichtung

## Patentansprüche

1. Druckgasmotor (1) aufweisend einen Arbeitskolben (12), einen Innenraum, der an einer Rückseite geschlossen ist und in dem der Arbeitskolben (12) linear beweglich angeordnet ist, ein Rückstellelement (22), das zumindest zeitweise eine in Richtung der Rückseite wirkende Kraft auf den Arbeitskolben (12) ausübt, eine Gaseinlassöffnung (24) zum Einspeisen eines unter Druck stehenden Gases in den Innenraum und eine Gasauslassöffnung (26) zum Abführen des Gases aus dem Innenraum, **dadurch gekennzeichnet, dass** zwischen dem Arbeitskolben (12) und der geschlossenen Rückseite ein Steuerkolben (14) linear beweglich im Innenraum angeordnet ist, wobei der Steuerkolben (14) in einer ersten Position die Gasauslassöffnung (26) abdeckt und die Gaseinlassöffnung (24) nicht abdeckt und in einer zweiten Position die Gaseinlassöffnung (24) abdeckt und die Gasauslassöffnung (26) nicht abdeckt, der Steuerkolben (14) gegen den Arbeitskolben (12) beweglich gelagert ist und an dem Arbeitskolben (12) und/oder dem Steuerkolben (14) ein Mitnehmerelement (18, 20, 42) angeordnet ist, das bei einer Bewegung des Arbeitskolbens (12) vom Steuerkolben (14) weg den Steuerkolben (14) in Richtung des Arbeitskolbens (12) mit einem ersten Abstand nachzieht.

2. Druckgasmotor (1) nach Anspruch 1, **dadurch gekennzeichnet, dass** der Steuerkolben (14) in einer dritten Position zwischen der ersten Position und der zweiten Position sowohl die Gaseinlassöffnung (24) als auch die Gasauslassöffnung (26) abdeckt.

3. Druckgasmotor (1) nach Anspruch 1 oder 2, **dadurch gekennzeichnet, dass** an der dem Arbeitskolben (12) zugewandten Seite des Steuerkolbens (14) zumindest ein Abstandhalter (16) angeordnet ist und/oder an der dem Steuerkolben (14) zugewandten Seite des Arbeitskolbens (12) zumindest ein Abstandhalter (16) angeordnet ist, so dass zwischen dem Arbeitskolben (12) und dem Steuerkolben (14) ein Zwischenraum entsteht, wenn diese aneinander anliegen, und die Gaseinlassöffnung (24) in der ersten Position in diesen Zwischenraum mündet, wobei durch den zumindest einen Abstandhalter (16) bei aneinander anliegenden Arbeitskolben (12) und Steuerkolben (14) ein zweiter Abstand zwischen dem Arbeitskolben (12) und dem Steuerkolben (14) eingestellt wird, der kleiner ist als der erste Abstand, der durch das Mitnehmerelement (18, 20, 42) eingestellt wird.

4. Druckgasmotor (1) nach einem der vorangehenden Ansprüche, **dadurch gekennzeichnet, dass**
das Rückstellelement (22) eine elastische Feder (22) ist, die im Innenraum zwischen dem Arbeitskolben (12) und einem vorderen Ende des Innenraums angeordnet ist, wobei das vordere Ende des Innenraums gegenüber der Rückseite des Innenraums angeordnet ist.

5. Druckgasmotor (1) nach einem der vorangehenden Ansprüche, **dadurch gekennzeichnet, dass**
der Steuerkolben (14) in der ersten Position von dem Rückstellelement (22) über den Arbeitskolben (12) an die Rückseite gedrückt oder gezogen ist, wobei die Gaseinlassöfifnung (24) in eine Öffnung oder den Zwischenraum zwischen dem Arbeitskolben (12) und dem Steuerkolben (14) mündet.

6. Druckgasmotor (1) nach einem der vorangehenden Ansprüche, **dadurch gekennzeichnet, dass**
in dem Steuerkolben (14) wenigstens ein gasdurchlässiger Durchgang (17) angeordnet ist, die die dem Arbeitskolben (12) zugewandte Vorderseite des Steuerkolbens (14) mit der der Rückseite des Innenraums zugewandten Rückseite des Steuerkolbens (14) verbindet.

7. Druckgasmotor (1) nach einem der vorangehenden Ansprüche, **dadurch gekennzeichnet, dass**
der Arbeitskolben (12) vollumfänglich an der Innenwand des Innenraums anliegt, bevorzugt über ein Dichtungselement (15) vollumfänglich, gasdicht und druckdicht an dem Innenraum anliegt.

8. Druckgasmotor (1) nach einem der vorangehenden Ansprüche, **dadurch gekennzeichnet, dass**
an der dem Steuerkolben (14) abgewandten Seite des Arbeitskolbens (12) eine Stange (40), insbesondere eine Pleuelstange (40) befestigt ist, die durch die Vorderseite des Innenraums aus dem Innenraum herausragt.

9. Druckgasmotor (1) nach Anspruch 8, **dadurch gekennzeichnet, dass**
die Stange (40) durch eine gasdichte Führung (41) durch die Vorderseite des Innenraums aus dem Innenraum geführt wird und dass der Innenraum zwischen der Vorderseite und dem Arbeitskolben (12) gasdicht und druckdicht geschlossen ist und eine Gasfederung als Rückstellelement bildet.

10. Druckgasmotor (1) nach einem der Ansprüche 1 bis 8, **dadurch gekennzeichnet, dass**
in der der Rückseite gegenüberliegenden Vorderseite des Innenraums eine Ausstoßöffnung (8) vorgesehen ist und in der Vorderseite und/oder in der seitlichen Wandung des Innenraums eine Flüssigkeitszufuhröffnung (28) angeordnet ist, die zumindest zeitweise nicht vom Arbeitskolben (12) abgedeckt ist und die im nicht abgedeckten Zustand zwischen dem Arbeitskolben (12) und der Vorderseite des Innenraums angeordnet ist.

11. Druckgasmotor (1) nach Anspruch 10, **dadurch gekennzeichnet, dass**
die Ausstoßöffnung (8) durch ein Ventilelement (10), insbesondere ein Lippenventil (10) mit der Umgebung verbunden ist, wobei das Ventilelement (10) bei einem ausreichenden Überdruck gegenüber dem Umgebungsdruck geöffnet und ansonsten geschlossen ist, und dass an der Flüssigkeitszufuhröffnung (28) ein Rohr oder eine Schlauch (70) mit einem Rückschlagventil (34) angeschlossen ist, das sich bei einem Unterdruck im Innenraum zwischen dem Arbeitskolben (12) und der Vorderseite des Innenraums öffnet und eine Flüssigkeitszufuhr in den Innenraum ermöglicht.

12. Druckgasmotor (1) nach einem der vorangehenden Ansprüche, **dadurch gekennzeichnet, dass**
das Mitnehmerelement (18, 20, 42) eine Schnur (42), ein Kabel (42), ein Faden, eine Kette oder eine elastische Feder ist, die am Arbeitskolben (12) und am Steuerkolben (14) befestigt ist oder das Mitnehmerelement (18, 20, 42) eine Stange (18), eine Schnur, ein Kabel, ein Faden, eine Kette oder eine elastische Feder aufweist, die am Arbeitskolben (12) oder am Steuerkolben (14) befestigt ist und an der ein Mitnehmer (20) befestigt ist, der in einen Vorsprung im Arbeitskolben (12) oder im Steuerkolben (14) greift, wobei bevorzugt das Mitnehmerelement (18, 20, 42) durch eine am Arbeitskolben (12) befestigte Stange (18) gebildet wird, die durch eine Durchführung im Steuerkolben (14) reicht und an der ein Mitnehmer (20) befestigt ist, der nicht durch die Durchführung im Steuerkolben (14) passt und der auf der Rückseite des Steuerkolbens (14) in diesen eingreift um den Steuerkolben (14) mitzuziehen, wenn der Arbeitskolben (12) dazu ausreichend weit von dem Steuerkolben (14) entfernt ist und sich in Richtung von diesem weg bewegt.

13. Druckgasmotor (1) nach einem der vorangehenden Ansprüche, **dadurch gekennzeichnet, dass**
der Innenraum zumindest bereichsweise zylindrisch ist oder im Bereich eines Arbeitsraums des Arbeitskolbens (12) oder im gesamten Hubraum des Arbeitskolbens (12) und des Steuerkolbens (14) zylindrisch ist.

14. Druckgasmotor (1) nach einem der vorangehenden Ansprüche, **dadurch gekennzeichnet, dass**
der Arbeitskolben (12) zwei unterschiedlich große Querschnittsflächen senkrecht zur Bewegungsrichtung des Arbeitskolbens (12) aufweist, wobei der Innenraum dazu passende Innenwände mit unterschiedlichen Querschnittsflächen aufweist und die Querschnittsfläche auf der, der Rückseite zugewandten Seite des Arbeitskolbens (12) kleiner ist als die Querschnittsfläche der gegenüberliegenden Vorderseite des Arbeitskolbens (12), bevorzugt die Querschnittsfläche auf der, der Rückseite zugewandten Seite des Arbeitskolbens (12) höchstens halb so groß wie die Querschnittsfläche der gegenüberliegenden Vorderseite des Arbeitskolbens (12) ist.

15. Lavage-System aufweisend zumindest einen Druckgasmotor (1) nach einem der vorangehenden Ansprüche, bei dem mit dem Druckgasmotor (1) oder den Druckgasmotoren (1) ein periodischer Sprühstoß mit einer Flüssigkeit erzeugbar ist.

16. Lavage-System nach Anspruch 15, **dadurch gekennzeichnet, dass** das Lavage-System einen Anschluss (56) für eine Druckgaspatrone (54), einen Druckminderer (60), einen Verdampfungsraum (58) zum Verdampfen von flüssigen Flüssiggasresten und ein manuell betätigbares Steuerungsventil (64) aufweist, wobei der Verdampfungsraum (58) über eine Druckgasleitung mit dem Anschluss (56) verbunden ist, der Druckminderer (60) über eine Druckgasleitung mit dem Verdampfungsraum (58) verbunden ist, das Steuerungsventil (64) über eine Druckgasleitung (68) mit dem Druckminderer (60) verbunden ist und die Gaseinlassöffnung (24) des Druckgasmotors (1) über eine Druckgasleitung (68) mit dem Steuerungsventil (64) verbunden ist und wobei vorzugsweise eine Flüssigkeitszufuhröffnung (28) des Druckgasmotors (1) mit einer Flüssigkeitsleitung (70) verbunden ist.

17. Verwendung eines Druckgasmotors (1) nach einem der Ansprüche 1 bis 14 als Motor für ein Lavage-System, einen Klopfermotor, als Motor für ein Spielzeug, einen Vibrationsmotor, als Antrieb für eine Dosiervorrichtung, als Rüttelmotor oder als Pumpe, insbesondere als Schmiermittelpumpe.

18. Verfahren zum Erzeugen einer periodischen Bewegung mit einem Druckgas, insbesondere unter Verwendung eines Druckgasmotors (1) nach einem der Ansprüche 1 bis 14, **dadurch gekennzeichnet, dass**
in einem Ausgangszustand der Arbeitskolben (12) und der Steuerkolben (14) in dem Innenraum voneinander mit dem ersten Abstand beabstandet werden, wobei der Steuerkolben (14) die Gasauslassöffnung (26) verschließt und die Gaseinlassöffnung (24) zwischen dem Arbeitskolben (12) und dem Steuerkolben (14) offen liegt;
das Druckgas zwischen den Arbeitskolben (12) und den Steuerkolben (14) in den Innenraum geleitet wird;
der Arbeitskolben (12) durch den Druck des Druckgases in Richtung einer Vorderseite des Innenraums vom Steuerkolben (14) weg beschleunigt und bewegt wird, wobei sich der Abstand zwischen dem Arbeitskolben (12) und dem Steuerkolben (14) vergrößert;
der Steuerkolben (14) über das Mitnehmerelement (18, 20, 42) von dem Arbeitskolben (12) mitgezogen wird, bevorzugt ab Erreichen des zweiten Abstands;
der Steuerkolben (14) die Gaseinlassöffnung (24) aufgrund der Bewegung des Steuerkolbens (14) verschließt;
der Steuerkolben (14) die Gasauslassöffnung (26) aufgrund der Bewegung des Steuerkolbens (14) öffnet;
der Arbeitskolben (12) durch das Rückstellelement (22) in Richtung der Rückseite des Innenraums beschleunigt wird;
das Druckgas zwischen dem Arbeitskolben (12) und dem Steuerkolben (14) durch wenigstens eine gasdurchlässige Öffnung durch den Steuerkolben (14) in den Raum zwischen der Rückseite des Steuerkolbens (14) und der Rückwand des Innenraums strömt;
das Druckgas in dem Raum zwischen der Rückseite des Steuerkolbens (14) und der Rückwand des Innenraums durch die geöffnete Gasauslassöffnung (26) an die Umgebung abgegeben wird;
der Arbeitskolben (12) auf den Steuerkolben (14) trifft und diesen in Richtung der Rückseite des Innenraums bewegt;
durch die umgekehrte Bewegung des Steuerkolbens (14) die Gasauslassöffnung (26) wieder geschlossen wird und
durch die umgekehrte Bewegung des Steuerkolbens (14) die Gaseinlassöffnung (24) wieder geöffnet wird, so dass erneut Druckgas zwischen den Arbeitskolben (12) und den Steuerkolben (14) in den Innenraum geleitet wird.

19. Verfahren nach Anspruch 18, **dadurch gekennzeichnet, dass** der Zyklus sich mit dem erneuten Einspeisen des Druckgases wiederholt.

20. Verfahren nach Anspruch 18 oder 19, **dadurch gekennzeichnet, dass**
der erste Abstand zwischen dem Arbeitskolben (12) und dem Steuerkolben (14) mit Hilfe zumindest eines Abstandhalters (16) eingestellt wird und der zweite Abstand zwischen dem Arbeitskolben (12) und dem Steuerkolben (14) mit einem Mitnehmerelement (18, 20, 42) eingestellt wird, vorzugsweise durch die Länge des Mitnehmerelements (18, 20, 42).

21. Verfahren zum Erzeugen eines Sprühstoßes aufweisend die Verfahrensschritte nach einem der Ansprüche 18 bis 20, **dadurch gekennzeichnet, dass**
bei einer Bewegung des Arbeitskolbens (12) weg von der Rückseite des Innenraums eine Flüssigkeit oder ein Flüssigkeit-Gas-Gemisch aus dem Raum zwischen dem Arbeitskolben (12) und der Vorderseite des Innenraums durch eine Ausstoßöffnung (8) an der Vorderseite des Innenraums hinaus gestoßen wird und
bei einer Bewegung des Arbeitskolbens (12) auf die Rückseite des Innenraums zu eine Flüssigkeit oder ein Flüssigkeit-Gas-Gemisch in den Raum zwischen dem Arbeitskolben (12) und der Vorderseite des Innenraums durch eine Flüssigkeitszufuhröffnung (28) hinein gedrückt oder gezogen wird.

22. Verfahren nach Anspruch 21, **dadurch gekennzeichnet, dass**
bei der Bewegung des Arbeitskolbens (12) auf die Vorderseite des Innenraums zu durch den Druck in dem Raum zwischen dem Arbeitskolben (12) und der Vorderseite des Innenraums ein Ventil (10) an der Ausstoßöffnung (8) geöffnet wird und/oder offen gehalten wird und ein an die Flüssigkeitszufuhröffnung (28) angeschlossenes Rückschlagventil (34) geschlossen wird und/oder geschlossen gehalten wird und
bei der Bewegung des Arbeitskolbens (12) auf die Rückseite des Innenraums zu durch den geringeren Druck in dem Raum zwischen dem Arbeitskolben (12) und der Vorderseite des Innenraums das Ventil (10) an der Ausstoßöffnung (8) geschlossen wird und/oder geschlossen gehalten wird und das an die Flüssigkeitszufuhröffnung (28) angeschlossene Rückschlagventil (34) geöffnet wird und/oder offen gehalten wird.
